(19) Europäisches Patentamt
European Patent Office
Office européen des brevets

(11) EP 4 671 258 A1

(12) # EUROPEAN PATENT APPLICATION
published in accordance with Art. 153(4) EPC

(43) Date of publication:
**31.12.2025 Bulletin 2026/01**

(21) Application number: **24759587.9**

(22) Date of filing: **07.02.2024**

(51) International Patent Classification (IPC):
***C07F 9/6558*** *(2006.01)* ***A61K 51/04*** *(2006.01)*
***A61K 31/675*** *(2006.01)* ***C07B 59/00*** *(2006.01)*
***A61P 35/04*** *(2006.01)* ***A61K 103/00*** *(2006.01)*
***A61K 103/20*** *(2006.01)* ***A61K 103/30*** *(2006.01)*
***A61K 103/40*** *(2006.01)*

(52) Cooperative Patent Classification (CPC):
Y02P 20/55

(86) International application number:
**PCT/CN2024/076669**

(87) International publication number:
**WO 2024/174910 (29.08.2024 Gazette 2024/35)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **21.02.2023 CN 202310140015**

(71) Applicant: **The Affiliated Hospital of Southwest Medical University**
**Luzhou, Sichuan 646000 (CN)**

(72) Inventors:
- **CHEN, Yue**
  **Luzhou, Sichuan 646000 (CN)**
- **XING, Naiguo**
  **Luzhou, Sichuan 646000 (CN)**
- **FENG, Yue**
  **Luzhou, Sichuan 646000 (CN)**
- **LI, Hongmei**
  **Luzhou, Sichuan 646000 (CN)**
- **WANG, Yudi**
  **Luzhou, Sichuan 646000 (CN)**
- **XU, Tingting**
  **Luzhou, Sichuan 646000 (CN)**
- **LIU, Huipan**
  **Luzhou, Sichuan 646000 (CN)**
- **CHEN, Zan**
  **Luzhou, Sichuan 646000 (CN)**
- **YANG, Jian**
  **Luzhou, Sichuan 646000 (CN)**
- **ZHAO, Yan**
  **Luzhou, Sichuan 646000 (CN)**
- **WANG, Li**
  **Luzhou, Sichuan 646000 (CN)**
- **ZHANG, Yu**
  **Luzhou, Sichuan 646000 (CN)**
- **WANG, Wei**
  **Luzhou, Sichuan 646000 (CN)**
- **CAI, Liang**
  **Luzhou, Sichuan 646000 (CN)**
- **HUANG, Zhanwen**
  **Luzhou, Sichuan 646000 (CN)**
- **ZHANG, Chunyin**
  **Luzhou, Sichuan 646000 (CN)**
- **QIU, Lin**
  **Luzhou, Sichuan 646000 (CN)**

(74) Representative: **Meissner Bolte Partnerschaft mbB**
**Patentanwälte Rechtsanwälte**
**Postfach 86 06 24**
**81633 München (DE)**

(54) **RADIOACTIVE LABEL OF RISEDRONIC ACID DERIVATIVE, AND PRECURSOR COMPOUND THEREOF, AND PREPARATION METHOD THEREFOR AND USE THEREOF**

(57) Disclosed in the present invention are a radiolabeled compound of a risedronic acid derivative, and a precursor compound thereof, and a preparation method therefor and the use, which belong to the technical field of nuclear medicine. Provided in the present invention are a precursor compound of a radiolabeled compound of a risedronic acid derivative as shown in formula (I) or a pharmaceutically acceptable salt thereof, and a radiolabeled compound of a risedronic acid derivative as shown in formula (II) or a pharmaceutically acceptable salt thereof. In the present invention, risedronic acid is creatively combined with a chelating agent to obtain NOTA-risedronic acid and DOTA-risedronic acid, and then a



Processed by Luminess, 75001 PARIS (FR)

**(Cont. next page)**

radionuclide is used for labelling. The radiolabeled product of the present invention has a relatively high water solubility, good in-vitro stability at room temperature, and a high plasma protein binding rate, and exhibits a relatively high and prolonged bone uptake in terms of imaging and in vivo distribution in mice; and compared to $^{68}$Ga-DOTA-ibandronic acid, the uptake of the compound of the present invention at the lesion site is twice as high.

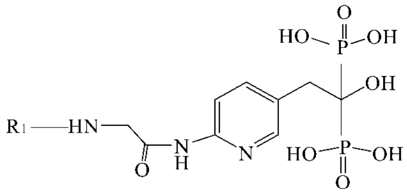

(I)

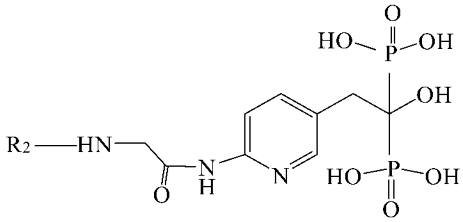

(II)

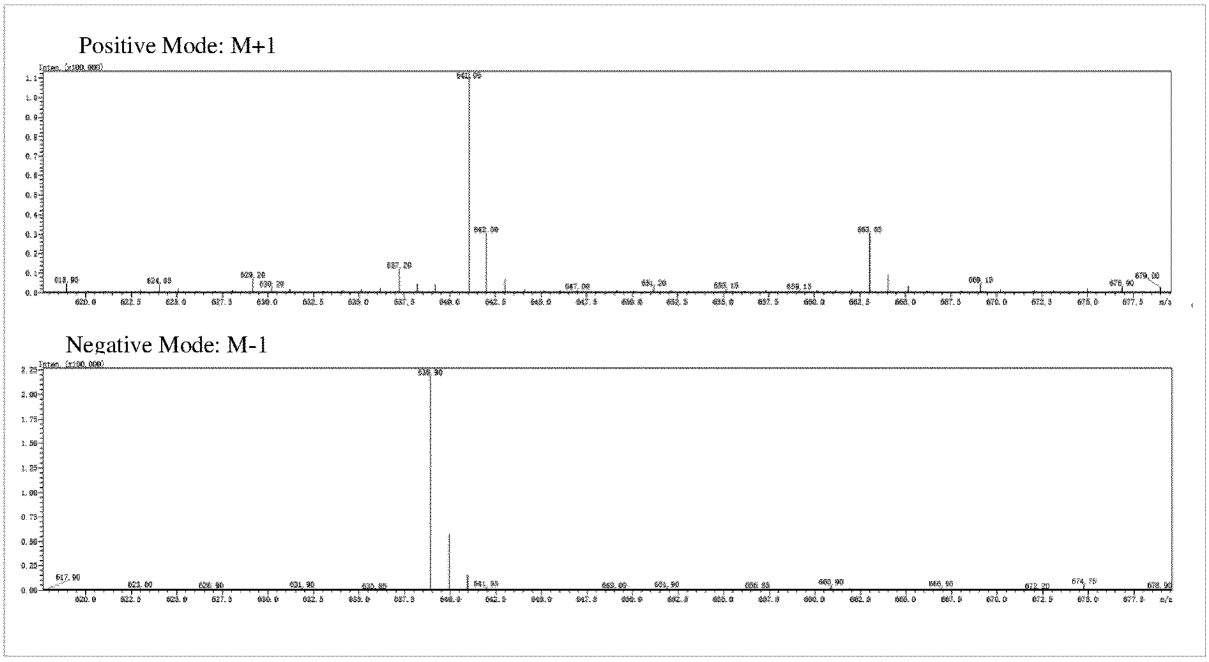


**Figure 1**

## Description

The present disclosure is based on the Chinese patent application with application number 202310140015.7 and application date February 21, 2023, and claims its priority. The content of the Chinese patent application is hereby incorporated into the present disclosure in its entirety.

## Technical Field

The present disclosure belongs to the field of nuclear medicine technology, and specifically relates to a radiolabeled compound of risedronic acid derivative, a precursor compound thereof, a preparation method therefor and use thereof.

## Background Art

Bone is a common site of distant metastasis of prostate cancer, breast cancer and lung cancer, and the spine is the most common site of bone metastasis. Studies have reported that 65% to 75% of breast cancer patients or prostate cancer patients and 30% to 40% of lung cancer patients may develop bone metastasis. Early diagnosis and treatment of metastatic bone tumors can effectively improve the prognosis and life quality of patients. Bone metastasis is often regarded as a sign of advanced disease, and the therapeutic regime is mostly palliative treatment. At the same time, skeletal-related events (SREs) caused by bone metastasis, such as bone pain, spinal cord compression, pathological fracture, hypercalcemia, etc., are the main factors affecting patients' ability to move independently and their life quality.

At present, the main treatments for metastatic bone tumors include chemotherapy, radiotherapy, endocrine therapy, surgical treatment, bisphosphonate therapy, and radionuclide therapy. Radionuclide therapy can significantly relieve bone pain, kill tumor cells, has few toxic and side effects, and is an effective and safe treatment method. At present, the drugs used in radionuclide therapy include $^{223}RaCl_2$, $^{89}SrCl_2$, and $^{153}$Sm-EDTMP, which can significantly relieve bone pain and reduce the incidence of skeletal-related events.

$^{68}$Ga, $^{111}$In, $^{89}$Zr, $^{177}$Lu, $^{225}$Ac, $^{64}$Cu, and $^{221}$At are all medical isotopes with excellent nuclear properties. $^{68}$Ga is obtained through $^{68}$Ge/$^{68}$Ga generator, which is simple to prepare and low in cost; it can emit $\beta^+$ rays for PET imaging. $^{111}$In, $^{89}$Zr, $^{177}$Lu, $^{225}$Ac, $^{64}$Cu, and $^{221}$At can all be purchased from abroad, with stable supply channels and sufficient supply.

$^{177}$Lu emits three kinds of $\beta^-$ particles with energy at 497keV (78.6%), 384keV (9.1%) and 176keV (12.2%), which can be used for treatment, and it also emits $\gamma$ rays at 113keV (6.4%) and 208keV (11%), with a half-life (T1/2) of 6.7d, which is suitable for in vivo localization imaging. The half-life (T1/2) of $^{111}$In is 2.3d, and the emitted rays can be used for both in vivo imaging and nuclide therapy. $^{89}$Zr is a new type of positronic nuclide with a half-life of 78.4h, and it first decays into an intermediate nuclide $^{89m}$Y through 22.3% of positron emission and 76.6% of electron capture, and then quickly decays into a stable nuclide $^{89}$Y (15.6s), releasing gamma photons at 909keV, which is suitable for in vivo localization imaging. $^{225}$Ac emits $\alpha$ rays. $\alpha$ particles have higher linear energy transfer, high ray energy, short range, higher relative biological effect, and the strongest killing effect on tumor cells. $^{225}$Ac has a half-life (T1/2) of 9.9d, and its daughter nuclide $^{213}$Bi has a half-life (T1/2) of 46min. The nuclide $^{64}$Cu has become a research hotspot in the field of nuclear medicine molecular probes and theranostic drugs due to its suitable half-life (12.7h), unique decay properties ($\beta^+$ decay, $\beta^-$ decay, electron capture), and ability to form complexes with a variety of ligands, etc. The average energy of $\alpha$ rays released by $^{211}$At decay is 6.8MeV, its half-life is 7.2h, and its range in tissue is 55-88 $\mu$m, which has strong clinical application values.

$^{99m}$Tc-MDP is the most commonly used bone imaging agent in clinical practice today, but as a single-photon imaging agent, its localization and display of lesions are far lower than positron imaging agents. Phosphonates (HEDP) are drugs commonly used in clinical studies for imaging and targeted therapy of metastatic bone tumors. However, as the first generation of nitrogen-free bisphosphonates, HEDP has significantly lower potency than that of second- and third-generation nitrogen-containing bisphosphonates, such as alendronic acid and risedronic acid. Therefore, exploring drugs with better imaging quality, better therapeutic effect, and abilities for both imaging and treatment of tumor bone metastasis has become an urgent problem to be solved by those skilled in the art.

## Contents of the present disclosure

A first object of the present disclosure is to provide a precursor compound of a radiolabeled compound of a risedronic acid derivative, represented by Formula I, or a pharmaceutically acceptable salt thereof, which has good imaging effect and can treat bone tumors after being labeled with nuclide.

A second object of the present disclosure is to provide a radiolabeled compound of a risedronic acid derivative, represented by Formula II, or a pharmaceutically acceptable salt thereof, which is obtained by nuclide labeling of a precursor compound represented by Formula I.

A third object of the present disclosure is to provide a method for preparing a precursor compound represented by Formula I.

A fourth object of the present disclosure is to provide a method for preparing a compound represented by Formula II.

A fifth object of the present disclosure is to provide a use of a precursor compound represented by Formula I.

A sixth object of the present disclosure is to provide a use of a compound represented by Formula II.

To achieve the above objects, the technical solutions adopted by the present disclosure are as follows:
In the first aspect, the present disclosure provides a precursor compound of radiolabeled compound of risedronic acid derivative, as represented by Formula I, or a pharmaceutically acceptable salt thereof,

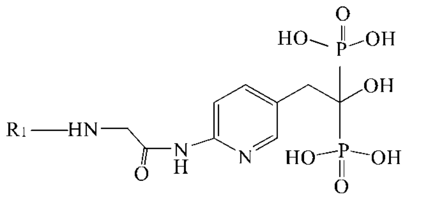

Formula I ,

wherein $R_1$ is

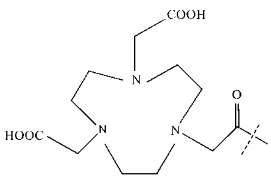
, or
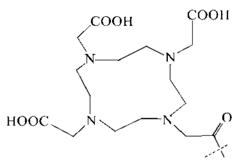
, or
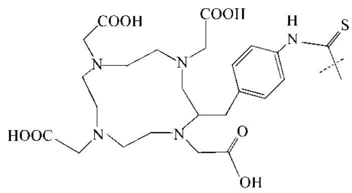
.

In the second aspect, the present disclosure provides a radiolabeled compound of risedronic acid derivative, as represented by Formula II, or a pharmaceutically acceptable salt thereof,

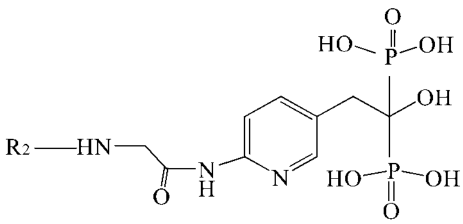

Formula II ,

wherein $R_2$ is

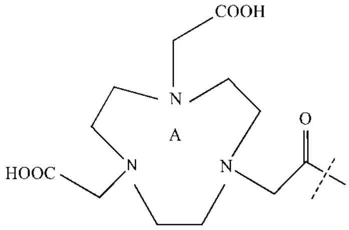
, or
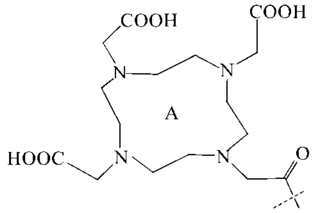
, or

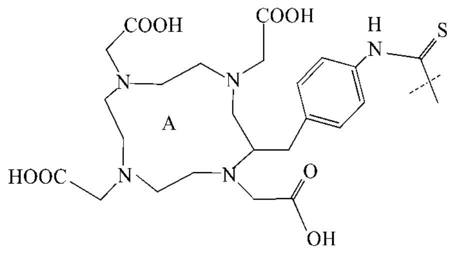

;

A is a radionuclide, preferably $^{68}Ga$, $^{111}In$, $^{89}Zr$, $^{177}Lu$, $^{225}Ac$, $^{64}Cu$ or $^{221}At$.

In some embodiments of the present disclosure, the compound represented by Formula II or pharmaceutically acceptable salt thereof has a radiochemical purity of greater than or equal to 95%.

In the third aspect, the present disclosure provides a method for preparing a precursor compound of radiolabeled compound, represented by Formula I, or a pharmaceutically acceptable salt thereof, which comprises the following steps:

Step 1, reacting Compound **1** with N-Boc glycine to generate Compound **2;**

Step 2, reacting Compound **2** to generate Compound **3** under the action of LiOH;

Step 3, adding chlorobenzene, $H_3PO_3$ and $POCl_3$ to Compound **3,** heating and reacting to generate Compound **4;**

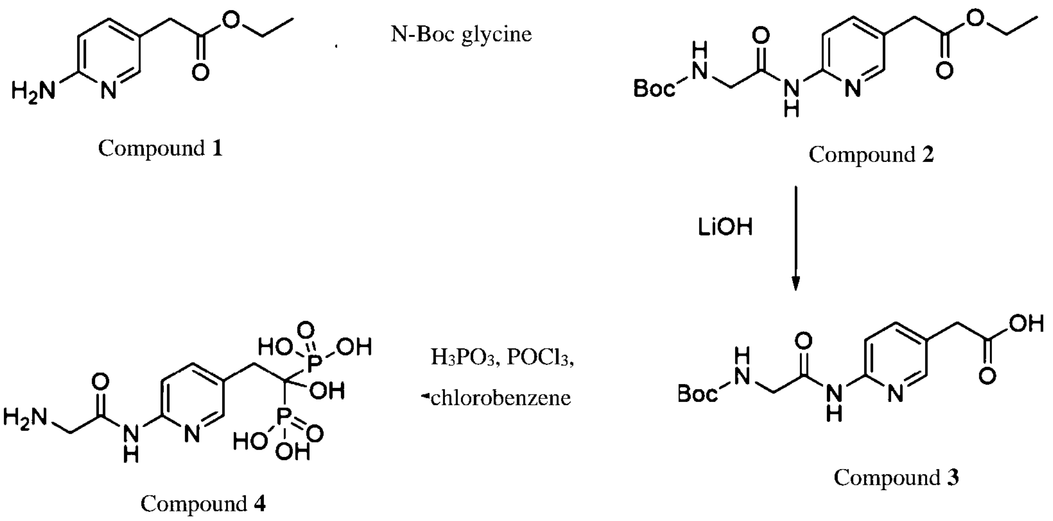


Compound **1**

Compound **2**

Compound **3**

Compound **4**

Step 4, when $R_1$ is

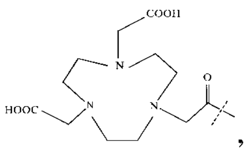

,

reacting Compound **4** with NOTA-NHS-ester to generate a compound represented by Formula I-1, wherein the reaction scheme is:

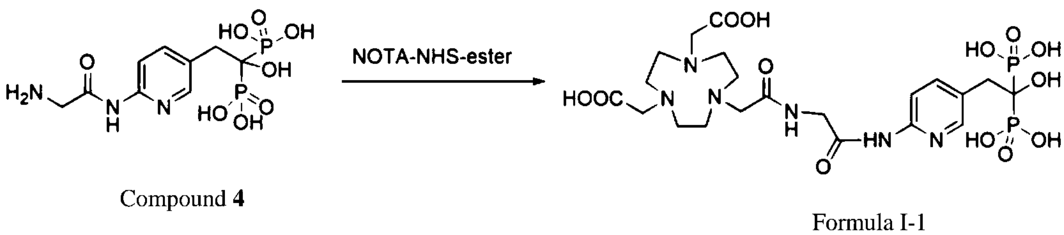


Compound **4**

Formula I-1

when $R_1$ is

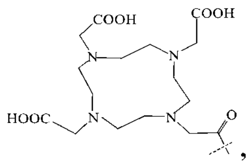

reacting Compound **4** with DOTA-NHS-ester to generate a compound represented by Formula I-2, wherein the reaction scheme is:

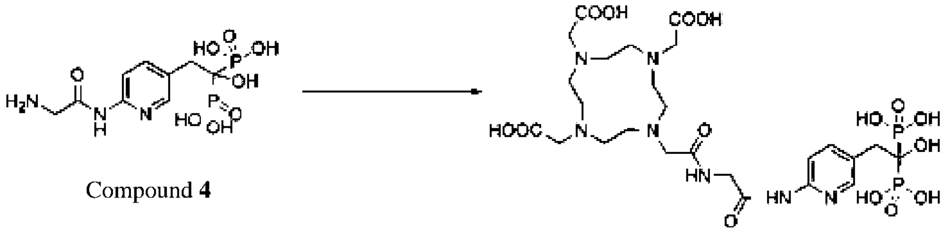

Compound **4**

Formula I-2

preferably,

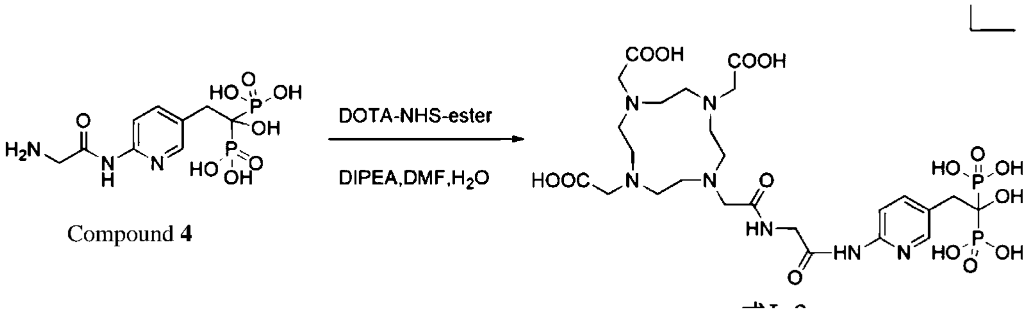


Compound **4**

Formula I-2

when $R_1$ is

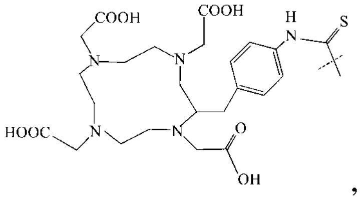

reacting Compound **4** with DOTA-p-Bn-NCS to generate a compound represented by Formula I-3, wherein the reaction scheme is:

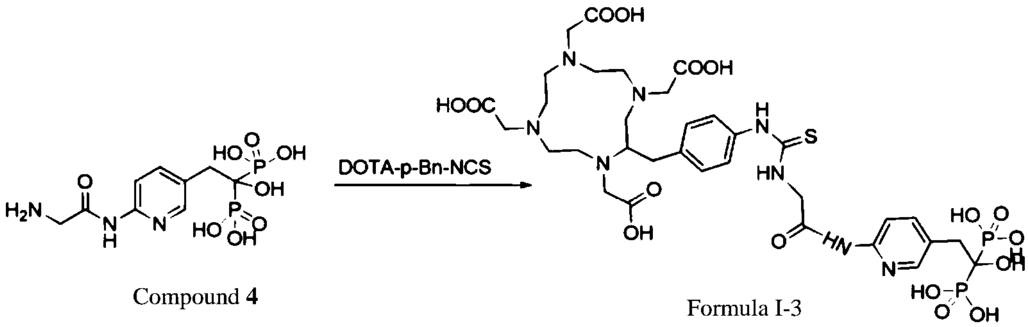


Compound **4**

Formula I-3

In the fourth aspect, the present disclosure provides a method for preparing a radiolabeled compound represented by Formula II, which comprises a step of reacting the compound represented by Formula I with a radionuclide salt solution to obtain the radiolabeled compound represented by Formula II.

In some embodiments of the present disclosure, the method comprises steps of mixing a solution of the compound represented by Formula I, a sodium salt solution and the radionuclide salt solution, adjusting the pH value of the obtained mixed solution, reacting the obtained solution, adjusting the pH value of the resulting reaction product, and filtering the resulting product to obtain the radiolabeled compound represented by Formula II.

In some embodiments of the present disclosure, when the radionuclide is $^{68}$Ga, $^{111}$In, $^{89}$Zr, $^{177}$Lu or $^{64}$Cu, the method comprises steps of mixing a solution of the compound represented by Formula I, a sodium acetate solution, and the radionuclide salt solution, adjusting pH value of the obtained mixed solution, reacting the obtained solution, adjusting pH value of the resulting reaction product, sterilizing the resulting product, and filtering to obtain the radiolabeled compound;

when the radionuclide is $^{225}$Ac, the method comprises steps of mixing a solution of the compound represented by Formula I, a sodium citrate solution, a sodium ascorbate solution, and the radionuclide salt solution, adjusting pH value of the obtained mixed solution, reacting the obtained solution, adjusting pH value of the resulting reaction product, sterilizing the resulting product, and filtering to obtain the radiolabeled compound;

when the radionuclide is $^{211}$At, the method comprises steps of mixing a solution of the compound represented by Formula I, a sodium borate solution, and the radionuclide salt solution, adjusting pH value of the obtained mixed solution, reacting the obtained solution, adjusting pH value of the resulting reaction product, sterilizing the resulting product, and filtering to obtain the radiolabeled compound.

In some embodiments of the present disclosure, the method comprises a step of reacting the compound represented by Formula I-1 with the radionuclide salt solution, specifically comprises the following steps:

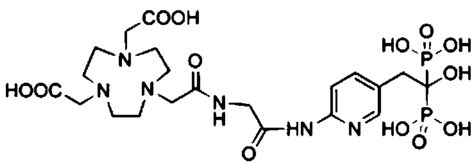

Formula I-1

when the radionuclide is $^{68}$Ga, adding 0.8-1.5ml of 0.25M sodium acetate solution to a solution of the compound represented by Formula I-1 with a solute content of 30-40$\mu$g, then adding a $^{68}$Ga salt solution with an activity of 10mCi, and mixing; adjusting the pH value of the obtained mixed solution to 4-7, preferably 5; reacting the obtained solution at 80-100°C, preferably 95°C, with a reaction time of 10-30min, preferably 15min; after the reaction, adjusting the pH value of the resulting reaction product to 5; wherein the solution of the compound represented by Formula I-1 has a concentration of 1mg/ml;, and the $^{68}$Ga salt solution has a concentration of 5mCi/ml to 10mCi/ml;

when the radionuclide is $^{111}$In, adding 0.8-1.5ml of 0.25M sodium acetate solution to a solution of the compound represented by Formula I-1 with a solute content of 30-40$\mu$g, then adding a $^{111}$In salt solution with an activity of 10mCi, and mixing; adjusting the pH value of the obtained mixed solution to 4-7, preferably 5.5; reacting the obtained solution at 80-100°C, preferably 95°C, with a reaction time of 10-30min, preferably 15min; after the reaction, adjusting the pH value of the resulting reaction product to 5; wherein the solution of the compound represented by Formula I-1 has a

concentration of 1mg/ml, and the $^{111}In$ salt solution has a concentration of 10mCi/ml to 20mCi/ml;

when the radionuclide is $^{89}Zr$, adding 0.8-1.5ml of 0.25M sodium acetate solution to a solution of the compound represented by Formula I-1 with a solute content of 30-40μg, then adding a $^{89}Zr$ salt solution with an activity of 2mCi, and mixing; adjusting the pH value of the obtained mixed solution to 4-7, preferably 5; reacting the obtained solution at 80-100°C, preferably 80°C, with a reaction time of 10-30min, preferably 15min; after the reaction, adjusting the pH value of the resulting reaction product to 4.5; wherein the solution of the compound represented by Formula I-1 has a concentration of 1mg/ml, and the $^{89}Zr$ salt solution has a concentration of 10mCi/ml to 20mCi/ml;

when the radionuclide is $^{177}Lu$, adding 0.8-1.5 ml of 0.25M sodium acetate solution to a solution of the compound represented by Formula I-1 with a solute content of 30-40μg, then adding a $^{177}Lu$ salt solution with an activity of 20mCi, and mixing; adjusting the pH value of the obtained mixed solution to 4-7, preferably 5; reacting the obtained solution at 80-100°C, preferably 95°C, with a reaction time of 10-30min, preferably 15min; after the reaction, adjusting the pH value of the resulting reaction product to 4.5; wherein the solution of the compound represented by Formula I-1 has a concentration of 1mg/ml, and the $^{177}Lu$ salt solution has a concentration of 20mCi/ml to 30mCi/ml;

when the radionuclide is $^{225}Ac$, adding 0.8-1.5 ml of 0.1M sodium ascorbate solution and 0.8-1.5 ml of 0.1M sodium citrate solution to a solution of the compound represented by Formula I-1 with a solute content of 20-30μg, then adding a $^{225}Ac$ salt solution with an activity of 0.01mCi, and mixing; adjusting the pH value of the obtained mixed solution to 4-7, preferably 5; reacting the obtained solution at 80-100°C, preferably 95°C, with a reaction time of 10-30min, preferably 15min; after the reaction, adjusting the pH value of the resulting reaction product to 5.5; wherein the solution of the compound represented by Formula I-1 has a concentration of 1mg/ml, and the $^{225}Ac$ salt solution has a concentration of 0.01mCi/ml to 0.02mCi/ml;

when the radionuclide is $^{64}Cu$, adding 0.8-1.5ml of 0.25M sodium acetate solution to a solution of the compound represented by Formula I-1 with a solute content of 30-40μg, then adding a $^{64}Cu$ salt solution with an activity of 5mCi, and mixing; adjusting the pH value of the obtained mixed solution to 4-7, preferably 5; reacting the obtained solution at 80-100°C, preferably 95°C, with a reaction time of 10-30min, preferably 15min; after the reaction, adjusting the pH value of the resulting reaction product to 4.5; wherein the solution of the compound represented by Formula I-1 has a concentration of 1mg/ml, and the concentration of the $^{64}Cu$ salt solution has a concentration of 5mCi/ml to 10mCi/ml;

when the radionuclide is $^{211}At$, adding 0.8-1.5ml of 0.25M sodium borate solution to a solution of the compound represented by Formula I-1 with a solute content of 30-40μg, then adding a $^{211}At$ salt solution with an activity of 1mCi, and mixing; adjusting the pH value of the obtained mixed solution to 4-7, preferably 5; reacting the obtained solution at 80-100°C, preferably 95°C, with a reaction time of 10-30min, preferably 15min; after the reaction, adjusting the pH value of the resulting reaction product to 6.5; wherein the solution of the compound represented by Formula I-1 has a concentration of 1mg/ml, and the $^{211}At$ salt solution has a concentration of 1.0mCi/ml to 2.0mCi/ml.

In some embodiments of the present disclosure, the method comprises a step of reacting the compound represented by Formula I-2 with the radionuclide salt solution, specifically comprises the following steps:

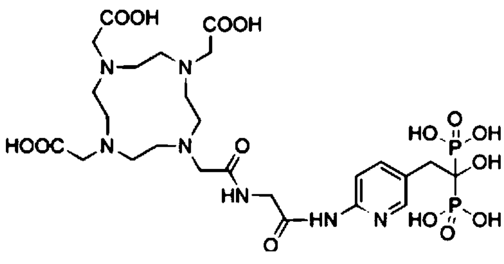

Formula I-2

when the radionuclide is $^{68}Ga$, adding 0.8-1.5ml of 0.25M sodium acetate solution to a solution of the compound represented by Formula I-2 with a solute content of 30-40μg, then adding a $^{68}Ga$ salt solution with an activity of 10mCi, and mixing; adjusting the pH value of the obtained mixed solution to 4-7, preferably 4.5; reacting the obtained solution at 80-100°C, preferably 95°C, with a reaction time of 10-30min, preferably 15min; after the reaction, adjusting the pH value of the resulting reaction product to 5; wherein the solution of the compound represented by Formula I-2 has a concentration of 1mg/ml, and the $^{68}Ga$ salt solution has a concentration of 5mCi/ml to 10mCi/ml;

when the radionuclide is $^{111}In$, adding 0.8-1.5ml of 0.25M sodium acetate solution to a solution of the compound represented by Formula I-2 with a solute content of 30-40μg, and then adding a $^{111}In$ salt solution with an activity of 10mCi, and mixing; adjusting the pH value of the obtained mixed solution to 4-7, preferably 5.5; reacting the obtained solution at 80-100°C, preferably 95°C, with a reaction time of 10-30min, preferably 15min; after the reaction, adjusting the pH value of the resulting reaction product to 4.5; wherein the solution of the compound represented by Formula I-2 has a concentration of 1mg/ml, and the $^{111}In$ salt solution has a concentration of 10mCi/ml to 20mCi/ml;

when the radionuclide is $^{89}Zr$, adding 0.8-1.5ml of 0.25M sodium acetate solution to a solution of the compound represented by Formula I-2 with a solute content of 30-40μg, and then adding a $^{89}Zr$ salt solution with an activity of 2mCi, and mixing; adjusting the pH value of the obtained mixed solution to 4-7, preferably 5; reacting the obtained solution at 80-100°C, preferably 80°C, with a reaction time of 10-30 min, preferably 15 min; after the reaction, adjusting the pH value of the resulting reaction product to 4.5; wherein the solution of the compound represented by Formula I-2 has a concentration of 1 mg/ml, and the $^{89}Zr$ salt solution has a concentration of 10 mCi/ml to 20 mCi/ml;

when the radionuclide is $^{177}Lu$, adding 0.8-1.5 ml of 0.25 M sodium acetate solution to a solution of the compound represented by Formula I-2 with a solute content of 30-40 μg, and then adding a $^{177}Lu$ salt solution with an activity of 20 mCi, and mixing; adjusting the pH value of the obtained mixed solution to 4-7, preferably 5; reacting the obtained solution at 80-100°C, preferably 85°C, with a reaction time of 10-30min, preferably 15min; after the reaction, adjusting the pH value of the resulting reaction product to 5; wherein the solution of the compound represented by Formula I-2 has a concentration of 1mg/ml, and the $^{177}Lu$ salt solution has a concentration of 20mCi/ml to 30mCi/ml;

when the radionuclide is $^{225}Ac$, adding 0.8-1.5ml of 0.1M sodium ascorbate solution and 0.8-1.5 ml of 0.1 M sodium citrate solution to a solution of the compound represented by Formula I-2 with a solute content of 20-30μg, then adding a $^{225}Ac$ salt solution with an activity of 0.01mCi, and mixing; adjusting the pH value of the obtained mixed solution to 4-7, preferably 5; reacting the obtained solution at 80-100°C, preferably 90°C, with a reaction time of 10-30min, preferably 15min; after the reaction, adjusting the pH value of the resulting reaction product to 5.5; wherein the solution of the compound represented by Formula I-2 has a concentration of 1mg/ml, and the concentration of the $^{225}Ac$ salt solution has a concentration of 0.01mCi/ml to 0.02mCi/ml;

when the radionuclide is $^{64}Cu$, adding 0.8-1.5 ml of 0.25 M sodium acetate solution to a solution of the compound represented by Formula I-2 with a solute content of 30-40μg, then adding a $^{64}Cu$ salt solution with an activity of 5 mCi, and mixing; adjusting the pH value of the obtained mixed solution to 4-7, preferably 5.5; reacting the obtained solution at 80-100°C, preferably 90°C, with a reaction time of 10-30 min, preferably 15 min; after the reaction, adjusting the pH value of the resulting reaction product to 4.5; wherein the solution of the compound represented by Formula I-2 has a concentration of 1 mg/ml, and the $^{64}Cu$ salt solution has a concentration of 5mCi/ml to 10mCi/ml;

when the radionuclide is $^{211}At$, adding 0.8-1.5ml of 0.25M sodium borate solution to a solution of the compound represented by Formula I-2 with a solute content of 30-40μg, then adding a $^{211}At$ salt solution with an activity of 1mCi, and mixing; adjusting the pH value of the obtained mixed solution to 4-7, preferably 5; reacting the obtained solution at 80-100°C, preferably 95°C, with a reaction time of 10-30min, preferably 15min; after the reaction, adjusting the pH value of the resulting reaction product to 6.5; wherein the solution of the compound represented by Formula I-2 has a concentration of 1mg/ml, and the $^{211}At$ salt solution has a concentration of 1.0mCi/ml to 2.0mCi/ml.

In some embodiments of the present disclosure, the method comprises a step of reacting the compound represented by Formula I-3 with the radionuclide salt solution, specifically comprises the following steps:

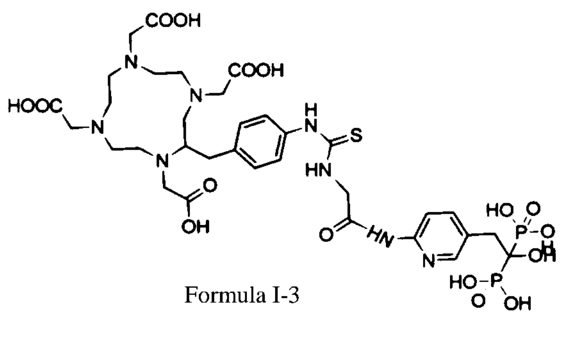

Formula I-3

when the radionuclide is $^{68}Ga$, adding 0.8-1.5ml of 0.25M sodium acetate solution to a solution of the compound

represented by Formula I-3 with a solute content of 20-40μg, then adding a $^{68}$Ga salt solution with an activity of 10mCi, and mixing; adjusting the pH value of the obtained mixed solution to 4-7, preferably 5; reacting the obtained solution at 80-100°C, preferably 85°C, with a reaction time of 10-30min, preferably 15min; after the reaction, adjusting the pH value of the resulting reaction product to 5; wherein the solution of the compound represented by Formula I-3 has a concentration of 1mg/ml, and the $^{68}$Ga salt solution has a concentration of 5mCi/ml to 10mCi/ml;

when the radionuclide is $^{111}$In, adding 0.7-1.4ml of 0.25M sodium acetate solution to a solution of the compound represented by Formula I-3 with a solute content of 30-40μg, then adding a $^{111}$In salt solution with an activity of 10mCi, and mixing; adjusting the pH value of the obtained mixed solution to 4-7, preferably 5.5; reacting the obtained solution at 80-100°C, preferably 90°C, with a reaction time of 10-30 min, preferably 15min; after the reaction, adjusting the pH value of the resulting reaction product to 5; wherein the solution of the compound represented by Formula I-3 has a concentration of 1mg/ml, and the $^{111}$In salt solution has a concentration of 10mCi/ml to 20mCi/ml;

when the radionuclide is $^{89}$Zr, adding 0.8-1.5ml of 0.25M sodium acetate solution to a solution of the compound represented by Formula I-3 with a solute content of 30-40μg, then adding a $^{89}$Zr salt solution with an activity of 2mCi, and mixing; adjusting the pH value of the obtained mixed solution to 4-7, preferably 5; reacting the obtained solution at 80-100°C, preferably 80°C, with a reaction time of 10-30min, preferably 15min; after the reaction, adjusting the pH value of the resulting reaction product to 4.5; wherein the solution of the compound represented by Formula I-3 has a concentration of 1mg/ml, and the $^{89}$Zr salt solution has a concentration of 10mCi/ml to 20mCi/ml;

when the radionuclide is $^{177}$Lu, adding 0.8-1.5ml of 0.25M sodium acetate solution to a solution of the compound represented by Formula I-3 with a solute content of 30-40μg, then adding a $^{177}$Lu salt solution with an activity of 20mCi, and mixing; adjusting the pH value of the obtained mixed solution to 4-7, preferably 5; reacting the obtained solution at 80-100°C, preferably 95°C, with a reaction time of 10-30min, preferably 15min; after the reaction, adjusting the pH value of the resulting reaction product to 4.5; wherein the solution of the compound represented by Formula I-3 has a concentration of 1mg/ml, and the $^{177}$Lu salt solution has a concentration of 20mCi/ml to 30mCi/ml;

when the radionuclide is $^{225}$Ac, adding 0.8-1.5ml of 0.1M sodium ascorbate solution and 0.8-1.5ml of 0.1M sodium citrate solution to a solution of the compound represented by Formula I-3 with a solute content of 20-30μg, then adding a $^{225}$Ac salt solution with an activity of 0.01mCi, and mixing; adjusting the pH value of the obtained mixed solution to 4-7, preferably 5; reacting the obtained solution at 80-100°C, preferably 95°C, with a reaction time of 10-30min, preferably 15min; after the reaction, adjusting the pH value of the resulting reaction product to 5.5; wherein the solution of the compound represented by Formula I-3 has a concentration of 1mg/ml, and the $^{225}$Ac salt solution has a concentration of 0.01mCi/ml to 0.02mCi/ml;

when the radionuclide is $^{64}$Cu, adding 0.8-1.5 ml of 0.25M sodium acetate solution to a solution of the compound represented by Formula I-3 with a solute content of 30-40μg, then adding a $^{64}$Cu salt solution with an activity of 5 mCi, and mixing; adjusting the pH value of the obtained mixed solution to 4-7, preferably 5; reacting the obtained solution at 80-100°C, preferably 95°C, with a reaction time of 10-30 min, preferably 15 min; after the reaction, adjusting the pH value of the resulting reaction product to 4.5; wherein the solution of the compound represented by Formula I-3 has a concentration of 1 mg/ml, and the $^{64}$Cu salt solution has a concentration of 5 mCi/ml to 10 mCi/ml;

when the radionuclide is $^{211}$At, adding 0.8-1.5 ml of 0.25 M sodium borate solution to a solution of the compound represented by Formula I-3 with a solute content of 30-40μg, then adding a $^{211}$At salt solution with an activity of 1 mCi, and mixing; adjusting the pH value of the obtained mixed solution to 4-7, preferably 5; reacting the obtained solution at 80-100°C, preferably 95°C, with a reaction time of 10-30 min, preferably 15 min; after the reaction, adjusting the pH value of the resulting reaction product to 6.5; wherein the solution of the compound represented by Formula I-3 has a concentration of 1 mg/ml, and the $^{211}$At salt solution has a concentration of 1.0 mCi/ml to 2.0 mCi/ml.

In the fifth aspect, the present disclosure provides a use of the precursor compound or pharmaceutically acceptable salt thereof of the first aspect of the present disclosure, or the radiolabeled compound or pharmaceutically acceptable salt thereof of the second aspect of the present disclosure in the manufacture of a medicament, especially in the manufacture of a medicament for imaging and/or treating a metastatic bone tumor.

In the sixth aspect, the present disclosure provides a method for imaging and/or treating a metastatic bone tumor, comprising: administering an effective amount of the radiolabeled compound or pharmaceutically acceptable salt thereof of the second aspect of the present disclosure to a subject in need.

The precursor compound or pharmaceutically acceptable salt thereof of the first aspect of the present disclosure, or the radiolabeled compound or pharmaceutically acceptable salt thereof of the second aspect of the present disclosure,

for use in imaging and/or treating a metastatic bone tumor.

Compared with the prior art, the present disclosure has the following beneficial effects:

The method of the present disclosure is simple and reasonably designed. The present disclosure creatively combines a risedronic acid with a chelating agent to obtain NOTA-risedronic acid or DOTA-risedronic acid, and then uses radionuclides $^{68}Ga$, $^{111}In$, $^{89}Zr$, $^{177}Lu$, $^{225}Ac$, $^{64}Cu$, or $^{221}At$ to label NOTA-risedronic acid or DOTA-risedronic acid to obtain $^{68}Ga$, $^{111}In$, $^{89}Zr$, $^{177}Lu$, $^{225}Ac$, $^{64}Cu$, $^{221}At$-NOTA-risedronic acids or $^{68}Ga$, $^{111}In$, $^{89}Zr$, $^{177}Lu$, $^{225}Ac$, $^{64}Cu$, $^{221}At$-DOTA-risedronic acids.

The radiolabeled product of the present disclosure has high water solubility, good in-vitro stability at room temperature, high plasma protein binding rate, and shows high and long lasting bone uptake in imaging and in-vivo distribution in mice, and thus it is a bone imaging agent and a therapeutic radiopharmaceutical for metastatic bone tumors with excellent performance.

The labeling method for preparing $^{68}Ga$, $^{111}In$, $^{89}Zr$, $^{177}Lu$, $^{225}Ac$, $^{64}Cu$, $^{221}At$-labeled NOTA-risedronic acid and DOTA-risedronic acid of the present disclosure is simple, has high labeling yield, short reaction time, small amount of precursor (microgram level), and high ratio of lesion target to non-target (T/N value) of up to 10 times or more. According to literature reports, a drug with a T/N ratio greater than 4-5 has a high therapeutic potential value. In addition, compared with $^{68}Ga$-DOTA-ibandronic acid, the compound of the present disclosure exhibits a two-fold uptake at lesion site, and the present disclosure has unexpected technical effects.

The names corresponding to the English abbreviations in the present disclosure are:

DCM: dichloromethane

PyBOP: benzotriazol-1-yl-oxytripyrrolidinophosphonium hexafluorophosphate

DIPEA: N-ethyldiisopropylamine

NOTA-NHS-ester: NOTA-succinimide ester

DMF: N,N-dimethylformamide

DOTA-NHS-ester: DOTA-succinimide ester

DOTA-p-Bn-NCS: 2-[(4-isothiocyanatophenyl)methyl]-1,4,7,10-tetraazacyclododecane-1,4,7,10-tetraacetic acid

## Brief Description of the Drawings

Figure 1 shows the LC-MS spectrum of the compound represented by Formula I-1;

Figure 2 shows the LC-MS spectrum of the compound represented by Formula 1-2;

Figure 3 shows the LC-MS spectrum of the compound represented by Formula I-3;

Figure 4 shows the TLC image of the $^{68}Ga$-labeled compound represented by Formula II-1;

Figure 5 shows the TLC image of the $^{177}Lu$-labeled compound represented by Formula II-1;

Figure 6 shows the TLC image of the $^{68}Ga$-labeled compound represented by Formula II-2;

Figure 7 shows the TLC image of the $^{177}Lu$-labeled compound represented by Formula II-2;

Figure 8 shows the TLC image of the $^{68}Ga$-labeled compound represented by Formula II-3;

Figure 9 shows the TLC image of the $^{177}Lu$-labeled compound represented by Formula II-3;

Figure 10 shows the PET/CT image of nude mice 2 hours after tail vein injection of $^{68}Ga$-DOTA-risedronic acid (the $^{68}Ga$-labeled compound represented by Formula II-1) in Test Example 1;

Figure 11 shows the PET/CT image of tumor-bearing mice treated with the $^{68}$Ga-labeled compound represented by Formula II-1 in Text Example 2;

Figure 12 shows the PET/CT image of tumor-bearing mice treated with $^{68}$Ga-DOTA-ibandronic acid in Test Example 2.

**Specific Models for Carrying Out the present disclosure**

In order to make the purpose, technical solution and advantages of the examples of the present disclosure clearer, the technical solution in the examples of the present disclosure will be described clearly and completely below. If the specific conditions were not specified in the examples, they were carried out under conventional conditions or the conditions recommended by the manufacturer. If the manufacturer of the reagents or instruments used in the examples was not specified, they were all conventional products that could be purchased commercially.

Example 1

In this example, a method for preparing Compound 4 was disclosed, specifically comprising:

S1, 180 mg (1 mmol) of Compound 1 and 210 mg (1.2 mmol) of N-Boc glycine were taken, added with 5 mL of DCM and stirred well, added with 780 mg (1.5 mmol) of PyBOP and 260 mg (2 mmol) of DIPEA, and reacted under stirring at room temperature for 24 hours. After TLC detection showed that Compound **1** had reacted completely, the reaction solution was diluted with DCM, washed with water and saturated brine in turn, dried over anhydrous sodium sulfate, concentrated to dryness, and separated by column chromatography (volume ratio of petroleum ether: ethyl acetate = 2:1) to obtain 300 mg of the target Compound **2;**

S2, 168 mg (0.5 mmol) of Compound **2** was taken, added with 1 mL of ethanol, 0.5 mL of water and 24 mg (1 mmol) of LiOH, and reacted at room temperature for 5 hours. After TLC detection showed that Compound **2** reacted completely, the reaction mixture was adjusted to pH 6 with 2M HCl, and extracted with dichloromethane. The extract was concentrated to dryness to obtain the target Compound **3,** which could be used for the next step without purification;

S3, 100 mg (0.33 mmol) of Compound **3** was taken, added with 5 mL of chlorobenzene and 270 mg (3.3 mmol) of phosphorous acid, heated to 90 °C, stirred for 30 min, then added with 1 g (6.6 mmol) of phosphorus oxychloride dropwise, and reacted for 8 h; the supernatant in the reaction product was discarded, the remaining solid was added with 5 mL of water, heated to 90°C and reacted for 12 h; filtration was carried out while hot, 10 mL of methanol was added to the filtrate; after filtration, the filter cake was oven-dried to obtain the target Compound **4** as a white solid. The reaction scheme was as follows:

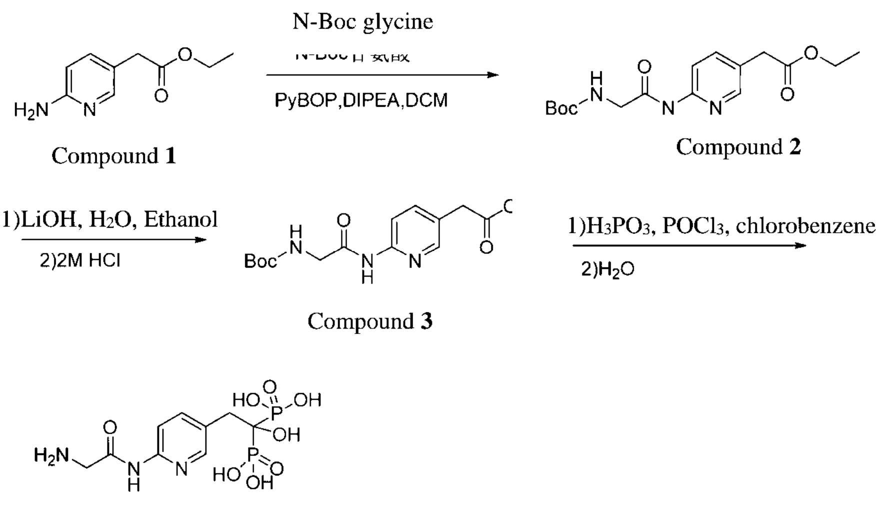


Compound **1**

Compound **2**

Compound **3**

Compound **4**

Example 2

In this example, a method for synthesizing the compound represented by Formula I-1 was disclosed, specifically comprising:
35 mg (0.1 mmol) of Compound **4** was taken, added with 99 mg (0.15 mmol) of NOTA-NHS-ester, added with 0.5 mL of DMF, 0.5 mL of water and 78 mg (0.6 mmol) of DIPEA, and reacted at room temperature for 24 h with LC-MS to monitor the product. The reaction solution was filtered and the filtrate was concentrated. The concentrate was purified by prep-HPLC to obtain the target compound represented by Formula I-1 as a white solid. The prep-HPLC operation conditions were: YMC-Actus Triart Diol-HILIC column, specification 150 mm×30 mm, 5 $\mu$m, 120 Å, and the mobile phase was 0.2 vol% acetic acid in acetonitrile. The reaction scheme was as follows:

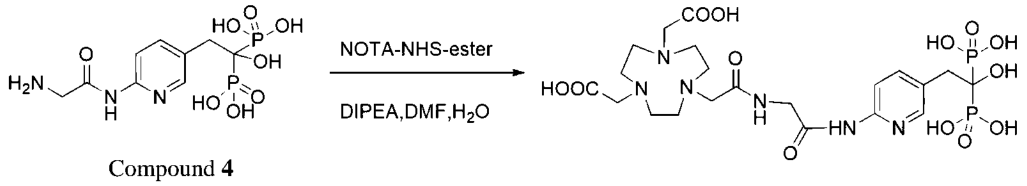


Compound **4**

Formula I-1

The LC-MS spectrum of the compound represented by Formula I-1 was shown in Figure 1.

Example 3

In this example, a method for synthesizing the compound represented by Formula I-2 was disclosed, specifically comprising:
35 mg (0.1 mmol) of Compound **4** was taken, added with 115 mg (0.15 mmol) of DOTA-NHS-ester, added with 0.5 mL of DMF, 0.5 mL of water and 78 mg (0.6 mmol) of DIPEA, and reacted at room temperature for 24 h with LC-MS to monitor the product. The reaction solution was filtered and the filtrate was concentrated. The concentrate was purified by prep-HPLC to obtain the target compound represented by Formula I-2 as a white solid. The prep-HPLC operation conditions were: YMC-Actus Triart Diol-HILIC column, specification 150 mm×30 mm, 5 $\mu$m, 120 Å, and the mobile phase was 0.2 vol% acetic acid in water. The reaction scheme was as follows:

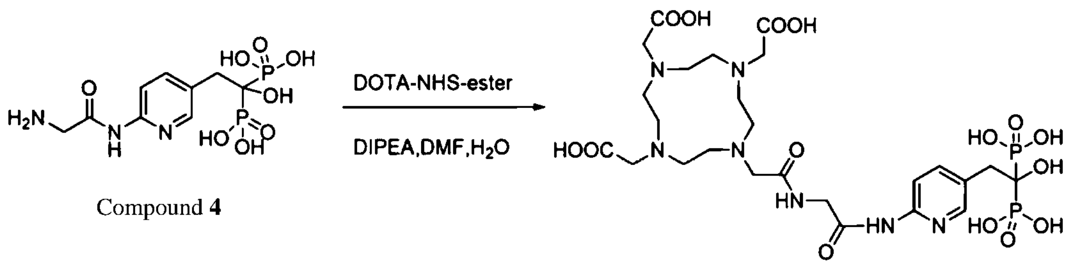


Compound **4**

Formula I-2

The LC-MS spectrum of the compound represented by Formula I-2 was shown in Figure 2.
The DOTA-NHS-ester in this example could be replaced by an equimolar amount of DOTA-p-Bn-NCS.

Example 4

In this example, a method for synthesizing the compound represented by Formula I-3 was disclosed, specifically comprising:
35 mg (0.1 mmol) of Compound **4** was taken, added with 83 mg (0.15 mmol) of DOTA-p-Bn-NCS, added with 0.5 mL of DMF, 0.5 mL of water and 78 mg (0.6 mmol) of DIPEA, and reacted at room temperature for 24 h with LC-MS to monitor the product. The reaction solution was filtered, and the filtrate was concentrated. The concentrate was purified by prep-HPLC to obtain the target compound represented by Formula I-3 as a white solid. The prep-HPLC operation conditions were: YMC-Actus Triart Diol-HILIC column, specification 150 mm×30 mm, 5$\mu$m, 120Å, and the mobile phase was 0.2 vol% acetic acid in water. The reaction scheme was as follows:

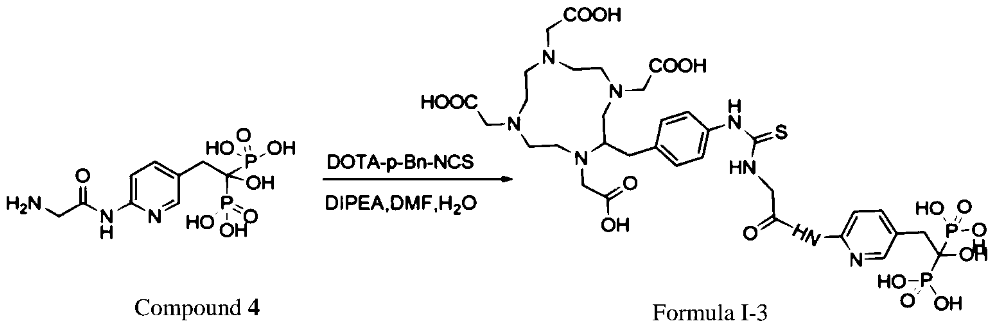


Compound **4**

Formula I-3

The LC-MS spectrum of the compound represented by Formula I-3 was shown in Figure 3.

Example 5

In this example, the reaction of the compound represented by Formula I-1 with a radionuclide salt solution to generate the compound represented by Formula II-1 was disclosed, and the reaction scheme was:

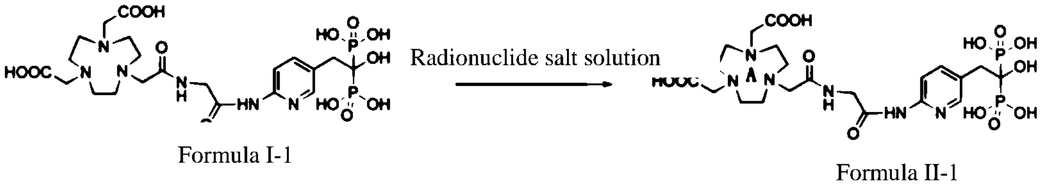


Formula I-1

Formula II-1

1. $^{68}$Ga as radionuclide

30μg of the compound represented by Formula I-1 was dissolved in sterile water to obtain a solution of the compound represented by Formula I-1 with a concentration of 1 mg/ml; 1.0 ml of 0.25M sodium acetate solution was added to the solution of the compound represented by Formula I-1, then 2ml of $^{68}$Ga salt solution with an activity of 10mCi was added, and mixed evenly; the pH value of the mixed solution was adjusted to 5, and the reaction was carried out at 80-100°C for 15min; after the reaction, the pH value of the reaction product was adjusted to 5 to obtain the $^{68}$Ga-labeled compound represented by Formula II-1, and its TLC image was shown in Figure 4.

2. $^{111}$In as radionuclide

30μg of the compound represented by Formula I-1 was dissolved in sterile water to obtain a solution of the compound represented by Formula I-1 with a concentration of 1 mg/ml; 1.0ml of 0.25M sodium acetate solution was added to the solution of the compound represented by Formula I-1, then 1ml of $^{111}$In salt solution with an activity of 10mCi was added, and mixed well; the pH value of the mixed solution was adjusted to 5.5, and the reaction was carried out at 95°C for 15min; after the reaction, the pH value of the reaction product was adjusted to 5 to obtain the $^{111}$In-labeled compound represented by Formula II-1.

3. $^{89}$Zr as radionuclide

30μg of the compound represented by Formula I-1 was dissolved in sterile water to obtain a solution of the compound represented by Formula I-1 with a concentration of 1 mg/ml; 1.0ml of 0.25M sodium acetate solution was added to the solution of the compound represented by Formula I-1, then 1ml of $^{89}$Zr salt solution with an activity of 2mCi was added, and mixed well; the pH value of the mixed solution was adjusted to 5, and the reaction was carried out at 80°C for 15min; after the reaction, the pH value of the reaction product was adjusted to 4.5 to obtain the $^{89}$Zr-labeled compound represented by Formula II-1.

4. $^{177}$Lu as radionuclide

30μg of the compound represented by Formula I-1 was dissolved in sterile water to obtain a solution of the compound represented by Formula I-1 with a concentration of 1 mg/ml; 1.0 ml of 0.25M sodium acetate solution was added to the solution of the compound represented by Formula I-1, then 2 ml of $^{177}$Lu salt solution with an activity of 20mCi was

added, and mixed evenly; the pH value of the mixed solution was adjusted to 5, and the reaction was carried out at 95°C for 15min; after the reaction, the pH value of the reaction product was adjusted to 4.5 to obtain the $^{177}$Lu-labeled compound represented by Formula II-1, and its TLC image was shown in Figure 5.

5. $^{225}$Ac as radionuclide

30μg of the compound represented by Formula I-1 was dissolved in sterile water to obtain a solution of the compound represented by Formula I-1 with a concentration of 1 mg/ml; 1.0ml of 0.1M sodium ascorbate solution and 1.0ml of 0.1M sodium citrate solution were added to the solution of the compound represented by Formula I-1, then 1ml of $^{225}$Ac salt solution with an activity of 0.01mCi was added, and mixed evenly; the pH value of the mixed solution was adjusted to 5, and the reaction was carried out at 95°C for 15min; after the reaction, the pH value of the reaction product was adjusted to 5.5 to obtain the $^{225}$Ac-labeled compound represented by Formula II-1.

6. $^{64}$Cu as radionuclide

30μg of the compound represented by Formula I-1 was dissolved in sterile water to obtain a solution of the compound represented by Formula I-1 with a concentration of 1 mg/ml; 1.0ml of 0.25M sodium acetate solution was added to the solution of the compound represented by Formula I-1, then 1ml of $^{64}$Cu salt solution with an activity of 5mCi was added, and mixed well; the pH value of the mixed solution was adjusted to 5, and the reaction was carried out at 95°C for 15min; after the reaction, the pH value of the reaction product was adjusted to 4.5 to obtain the $^{64}$Cu-labeled compound represented by Formula II-1.

7. $^{211}$At as radionuclide

30μg of the compound represented by Formula I-1 was dissolved in sterile water to obtain a solution of the compound represented by Formula I-1 with a concentration of 1 mg/ml; 1.0ml of 0.25M sodium borate solution was added to the solution of the compound represented by Formula I-1, then 1ml of $^{211}$At salt solution with an activity of 1mCi was added, and mixed evenly; the pH value of the mixed solution was adjusted to 5, and the reaction was carried out at 95°C for 15min; after the reaction, the pH value of the reaction product was adjusted to 6.5 to obtain the $^{211}$At-labeled compound represented by Formula II-1.

The radiochemical purity of the compound represented by Formula II-1 in this example was greater than or equal to 95%.

Example 6

In this example, the reaction of the compound represented by Formula I-2 with a radionuclide salt solution to generate the compound represented by Formula II-2 was disclosed, and the reaction scheme was:

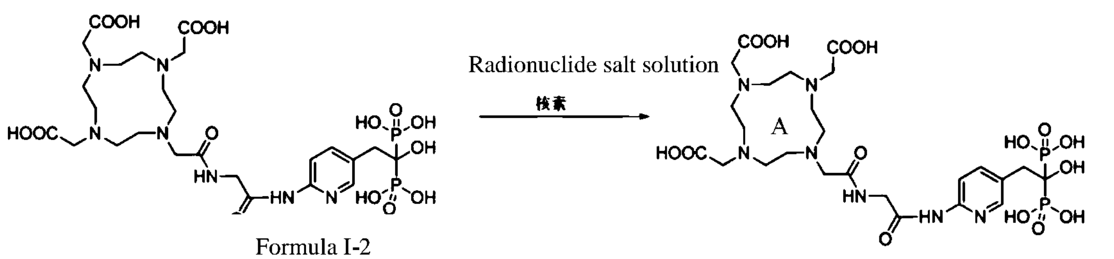


Formula I-2

Formula II-2 .

1. $^{68}$Ga as radionuclide

30μg of the compound represented by Formula I-2 was dissolved in sterile water to obtain a solution of the compound represented by Formula I-2 with a concentration of 1 mg/ml; 1.0ml of 0.25M sodium acetate solution was added to the solution of the compound represented by Formula I-2, then 2ml of $^{68}$Ga salt solution with an activity of 10mCi was added, and mixed evenly; the pH value of the mixed solution was adjusted to 4.5, and the reaction was carried out at 95°C for 15min; after the reaction, the pH value of the reaction product was adjusted to 5 to obtain the $^{68}$Ga-labeled compound represented by Formula II-2, and its TLC image was shown in Figure 6.

2. $^{111}$In as radionuclide

30μg of the compound represented by Formula I-2 was dissolved in sterile water to obtain a solution of the compound represented by Formula I-2 with a concentration of 1 mg/ml; 1.0ml of 0.25M sodium acetate solution was added to the solution of the compound represented by Formula I-2, then 1ml of $^{111}$In salt solution with an activity of 10mCi was added, and mixed evenly; the pH value of the mixed solution was adjusted to 5.5, and the reaction was carried out at 95°C for 15min; after the reaction, the pH value of the reaction product was adjusted to 4.5 to obtain the $^{111}$In-labeled compound represented by Formula II-2.

3. $^{89}$Zr as radionuclide

30μg of the compound represented by Formula I-2 was dissolved in sterile water to obtain a solution of the compound represented by Formula I-2 with a concentration of 1 mg/ml; 1.0ml of 0.25M sodium acetate solution was added to the solution of the compound represented by Formula I-2, then 1ml of $^{89}$Zr salt solution with an activity of 2mCi was added, and mixed well; the pH value of the mixed solution was adjusted to 5, and the reaction was carried out at 80°C for 15min; after the reaction, the pH value of the reaction product was adjusted to 4.5 to obtain the $^{89}$Zr-labeled compound represented by Formula II-2.

4. $^{177}$Lu as radionuclide

30μg of the compound represented by Formula I-2 was dissolved in sterile water to obtain a solution of the compound represented by Formula I-2 with a concentration of 1 mg/ml; 1.0ml of 0.25M sodium acetate solution was added to the solution of the compound represented by Formula I-2, then 2ml of $^{177}$Lu salt solution with an activity of 20mCi was added, and mixed evenly; the pH value of the mixed solution was adjusted to 5, and the reaction was carried out at 85°C for 15min; after the reaction, the pH value of the reaction product was adjusted to 5 to obtain the $^{177}$Lu-labeled compound represented by Formula II-2, and its TLC image was shown in Figure 7.

5. $^{225}$Ac as radionuclide

30μg of the compound represented by Formula I-2 was dissolved in sterile water to obtain a solution of the compound represented by Formula I-2 with a concentration of 1 mg/ml; 1.0ml of 0.1M sodium ascorbate solution and 1.0ml of 0.1M sodium citrate solution were added to the solution of the compound represented by Formula I-2, then 1ml of $^{225}$Ac salt solution with an activity of 0.01mCi was added, and mixed evenly; the pH value of the mixed solution was adjusted to 5, and the reaction was carried out at 90°C for 15min; after the reaction, the pH value of the reaction product was adjusted to 5.5 to obtain the $^{225}$Ac-labeled compound represented by Formula II-2.

6. $^{64}$Cu as radionuclide

30μg of the compound represented by Formula I-2 was dissolved in sterile water to obtain a solution of the compound represented by Formula I-2 with a concentration of 1 mg/ml; 1.0ml of 0.25M sodium acetate solution was added to the solution of the compound represented by Formula I-2, then 1ml of $^{64}$Cu salt solution with an activity of 5mCi was added, and mixed well; the pH value of the mixed solution was adjusted to 5.5, and the reaction was carried out at 90°C for 15min; after the reaction, the pH value of the reaction product was adjusted to 4.5 to obtain the $^{64}$Cu-labeled compound represented by Formula II-2.

7. $^{211}$At as radionuclide

30μg of the compound represented by Formula I-2 was dissolved in sterile water to obtain a solution of the compound represented by Formula I-2 with a concentration of 1 mg/ml; 1.0ml of 0.25M sodium borate solution was added to the solution of the compound represented by Formula I-2, then 1ml of $^{211}$At salt solution with an activity of 1mCi was added, and mixed evenly; the pH value of the mixed solution was adjusted to 5, and the reaction was carried out at 95°C for 15min; after the reaction, the pH value of the reaction product was adjusted to 6.5 to obtain the $^{211}$At-labeled compound represented by Formula II-2.

The radiochemical purity of the compound represented by Formula II-2 in this example was greater than or equal to 95%.

Example 7

In this example, the reaction of the compound represented by Formula I-3 with a radionuclide salt solution to generate the compound represented by Formula II-3 was disclosed, and the reaction scheme was:

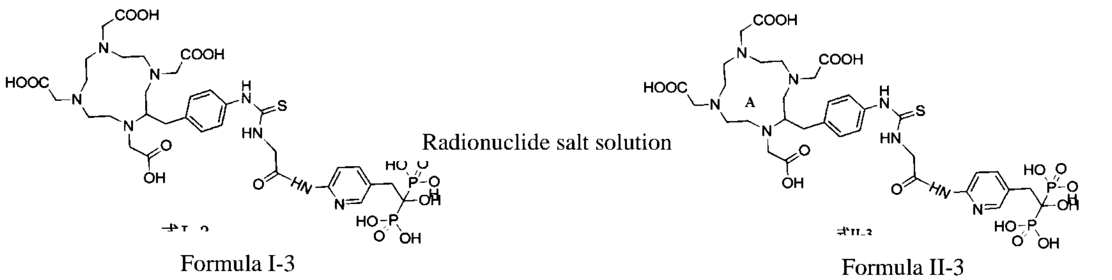


Formula I-3 Formula II-3

1. $^{68}Ga$ as radionuclide

40μg of the compound represented by Formula I-3 was dissolved in sterile water to obtain a solution of the compound represented by Formula I-3 with a concentration of 1 mg/ml; 1.0ml of 0.25M sodium acetate solution was added to the solution of the compound represented by Formula I-3, then 2ml of $^{68}Ga$ salt solution with an activity of 10mCi was added, and mixed evenly; the pH value of the mixed solution was adjusted to 5, and the reaction was carried out at 85°C for 15min; after the reaction, the pH value of the reaction product was adjusted to 5 to obtain the $^{68}Ga$-labeled compound represented by Formula II-3, and its TLC image was shown in Figure 8.

2. $^{111}In$ as radionuclide

40μg of the compound represented by Formula I-3 was dissolved in sterile water to obtain a solution of the compound represented by Formula I-3 with a concentration of 1 mg/ml; 1.0ml of 0.25M sodium acetate solution was added to the solution of the compound represented by Formula I-3, then 1ml of $^{111}In$ salt solution with an activity of 10mCi was added, and mixed evenly; the pH value of the mixed solution was adjusted to 5.5, and the reaction was carried out at 90°C for 15min; after the reaction, the pH value of the reaction product was adjusted to 5 to obtain the $^{111}In$-labeled compound represented by Formula II-3.

3. $^{89}Zr$ as radionuclide

40μg of the compound represented by Formula I-3 was dissolved in sterile water to obtain a solution of the compound represented by Formula I-3 with a concentration of 1 mg/ml; 1.0ml of 0.25M sodium acetate solution was added to the solution of the compound represented by Formula I-3, then 1ml of $^{89}Zr$ salt solution with an activity of 2mCi was added, and mixed evenly; the pH value of the mixed solution was adjusted to 5, and the reaction was carried out at 80°C for 15min; after the reaction, the pH value of the reaction product was adjusted to 4.5 to obtain the $^{89}Zr$-labeled compound represented by Formula II-3.

4. $^{177}Lu$ as radionuclide

40μg of the compound represented by Formula I-3 was dissolved in sterile water to obtain a solution of the compound represented by Formula I-3 with a concentration of 1 mg/ml; 1ml of 0.25M sodium acetate solution was added to the solution of the compound represented by Formula I-3, then 2ml of $^{177}Lu$ salt solution with an activity of 20mCi was added, and mixed evenly; the pH value of the mixed solution was adjusted to 5, and the reaction was carried out at 95°C for 15min; after the reaction, the pH value of the reaction product was adjusted to 4.5 to obtain the $^{177}Lu$ labeled compound represented by Formula II-3, and its TLC image was shown in Figure 9.

5. $^{225}Ac$ as radionuclide

30μg of the compound represented by Formula I-3 was dissolved in sterile water to obtain a solution of the compound represented by Formula I-3 with a concentration of 1 mg/ml; 1.0ml of 0.1M sodium ascorbate solution and 1.0ml of 0.1M sodium citrate solution were added to the solution of the compound represented by Formula I-3, and then 1ml of $^{225}Ac$ salt solution with an activity of 0.01mCi was added, and mixed evenly; the pH value of the mixed solution was

adjusted to 5, and the reaction was carried out at 95°C for 15min; after the reaction, the pH value of the reaction product was adjusted to 5.5 to obtain the $^{225}$Ac-labeled compound represented by Formula II-3.

6. $^{64}$Cu as radionuclide

40μg of the compound represented by Formula I-3 was dissolved in sterile water to obtain a solution of the compound represented by Formula I-3 with a concentration of 1 mg/ml; 1.0ml of 0.25M sodium acetate solution was added to the solution of the compound represented by Formula I-3, then 1ml of $^{64}$Cu salt solution with an activity of 5mCi was added, and mixed evenly; the pH value of the mixed solution was adjusted to 5, and the reaction was carried out at 95°C for 15min; after the reaction, the pH value of the reaction product was adjusted to 4.5 to obtain the $^{64}$Cu-labeled compound represented by Formula II-3.

7. $^{211}$At as radionuclide

40μg of the compound represented by Formula I-3 was dissolved in sterile water to obtain a solution of the compound represented by Formula I-3 with a concentration of 1 mg/ml; 1.0ml of 0.25M sodium borate solution was added to the solution of the compound represented by Formula I-3, then 1ml of $^{211}$At salt solution with an activity of 1mCi was added, and mixed evenly; the pH value of the mixed solution was adjusted to 5, and the reaction was carried out at 95°C for 15min; after the reaction, the pH value of the reaction product was adjusted to 6.5 to obtain the $^{211}$At-labeled compound represented by Formula II-3.

The radiochemical purity of the compound represented by Formula II-3 in this example was greater than or equal to 95%.

Test Example 1

In this test example, the PET/CT imaging experiment of the $^{68}$Ga-labeled compound represented by Formula II-1 of the present disclosure in tumor-bearing mice was disclosed.

(1) Establishment of tumor-bearing mouse model: 12 SPF female nude mice were prepared. After isoflurane anesthesia, the skin around the left knee joint was disinfected. The left hind limb was bent and an insulin needle (29G needle) was used to insert vertically into the femur from the concave point of the femoral head joint fossa. The needle was slowly rotated into the bone marrow cavity and 25μl (containing about $2\times10^6$ cells) of MDA-MB-231 (human breast cancer cells, from ATCC) cell culture medium was injected. After disinfection, the hole was sealed with sterile bone wax, and the animal was placed on a 37°C hot plate to rewarm. After recovery, it was returned to the cage for continued feeding.

(2) Verification of tumor-bearing mouse model: 4 weeks after inoculation, 12 nude mice were scanned using Micro-CT respectively. It was observed that all 12 nude mice had varying degrees of bone destruction and soft tissue swelling, indicating that the bone metastasis model was successfully established.

(3) Imaging of tumor-bearing mice: a successfully modeled tumor-bearing mouse was taken, injected with about 0.1 ml of the freshly prepared $^{68}$Ga-labeled compound represented by Formula II-1 (with a concentration of 1 mCi/ml) via the tail vein, and PET/CT whole-body imaging was performed 2 hours after injection. The results are shown in Figure 10. The whole-body bone imaging is clear 2 hours after injection, the joints of the limbs are clearly visualized, and the bone metastatic lesions at the left knee joint has obvious imaging agent uptake.

Test Example 2

In this test example, a comparative PET/CT imaging experiment of the $^{68}$Ga-labeled compound represented by Formula II-1 of the present disclosure and $^{68}$Ga-DOTA-ibandronic acid in tumor-bearing mice was disclosed. The $^{68}$Ga-DOTA-ibandronic acid was prepared according to the method No.5 in Table 1 in the description of the published Chinese patent application with application number 202111419244.X.

Four successfully modeled tumor-bearing mice were taken, in which two mice were injected with about 0.1 ml of the freshly prepared $^{68}$Ga-labeled compound represented by Formula II-1 (with a concentration of 1 mCi/ml) via the tail vein, and the other two mice were injected with about 0.1 ml of the freshly prepared $^{68}$Ga-DOTA-ibandronic acid (with a concentration of 1 mCi/ml) via the tail vein. PET/CT whole-body imaging was performed 2 hours after injection. The results were shown in Table 1 and Figures 11 and 12. Figure 11 showed the imaging of the tumor-bearing mice injected with the $^{68}$Ga-labeled compound represented by Formula II-1, and Figure 12 showed the imaging of the tumor-bearing mice

injected with the $^{68}Ga$-DOTA-ibandronic acid.

The T/N value was calculated according to the following formula:

$$T/N\ value = SUVmax\ of\ lesion/SUVmax\ of\ background\ tissue$$

Table 1: Imaging results of tumor-bearing mice

| | Head | | Liver | | Lesion | |
|---|---|---|---|---|---|---|
| | SUVmax | SUVmin | SUVmax | SUVmin | SUVmax | SUVmin |
| $^{68}Ga$-labeled compound represented by Formula 11-1 | 0.938 | 0.291 | 0.07 | 0.055 | 1.284 | 0.425 |
| $^{68}Ga$-DOTA-ibandronic acid | 0.783 | 0.230 | 0.067 | 0.046 | 0.647 | 0.139 |

The results show that the uptake of the $^{68}Ga$ labeled compound represented by Formula II-1 of the present disclosure is significantly higher than that of $^{68}Ga$-DOTA-ibandronic acid in both the head and lesion site, and the uptake in the lesion site is twice that of $^{68}Ga$-DOTA-ibandronic acid. The $^{68}Ga$-labeled compound represented by Formula II-1 of the present disclosure has an unexpectedly better imaging effect. Moreover, the ratio of lesion targets to non-targets (T/N value) of the $^{68}Ga$-labeled compound represented by Formula II-1 of the present disclosure is very high, up to 10 or more. According to literature reports, a T/N value of greater than 4-5 has a high therapeutic potential value. Hence, the $^{68}Ga$-labeled compound represented by Formula II-1 of the present disclosure has a high therapeutic potential value.

Finally, it should be noted that the above examples are only preferred examples of the present disclosure to illustrate the technical solution of the present disclosure, but not to limit it, and certainly not to limit the patent scope of the present disclosure. Any insubstantial changes or modifications made to the main design concept and spirit of the present disclosure should fall within the protection scope of the present disclosure, as long as they solve the same technical problems as those of the present disclosure; in addition, the direct or indirect application of the technical solution of the present disclosure in other related technical fields should also fall within the patent protection scope of the present disclosure.

## Claims

1. A precursor compound of radiolabeled compound of risedronic acid derivative, as represented by Formula I, or a pharmaceutically acceptable salt thereof,

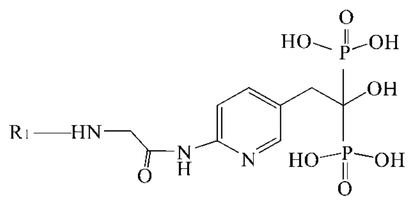

Formula I ,

wherein $R_1$ is

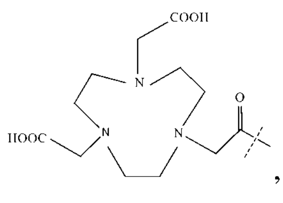, or 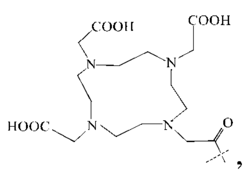, or 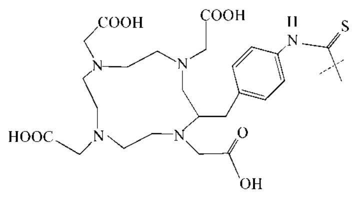.

2. A radiolabeled compound of risedronic acid derivative, as represented by Formula II, or a pharmaceutically

acceptable salt thereof,

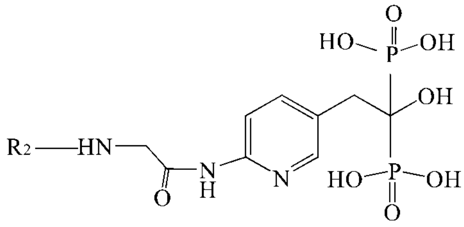

Formula II ,

wherein $R_2$ is

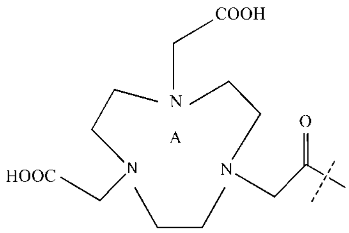

, or

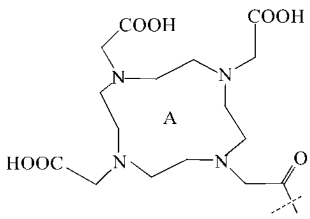

,

or

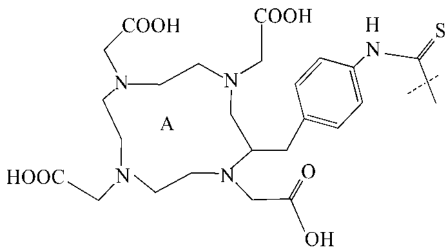

;

A is a radionuclide, preferably $^{68}Ga$, $^{111}In$, $^{89}Zr$, $^{177}Lu$, $^{225}Ac$, $^{64}Cu$ or $^{221}At$.

**3.** The radiolabeled compound or pharmaceutically acceptable salt thereof according to claim 2, which has a radiochemical purity of greater than or equal to 95%.

**4.** A method for preparing the precursor compound of radiolabeled compound or pharmaceutically acceptable salt thereof according to claim 1, which comprises the following steps:

Step 1, reacting Compound **1** with N-Boc glycine to generate Compound **2;**
Step 2, reacting Compound **2** to generate Compound **3** under the action of LiOH;
Step 3, adding chlorobenzene, $H_3PO_3$ and $POCl_3$ to Compound **3,** heating and reacting to generate Compound **4;**

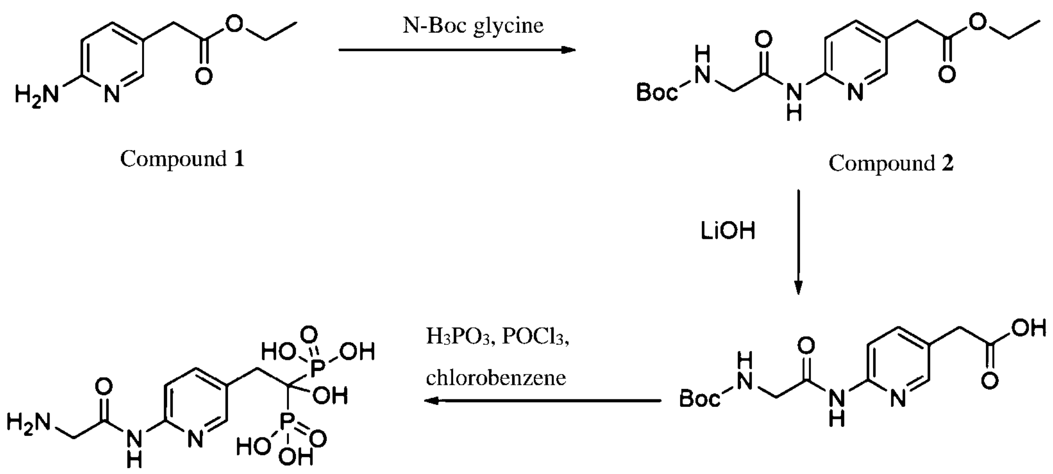


Compound **1**

Compound **2**

Compound **4**

Compound **3**

Step 4, when $R_1$ is

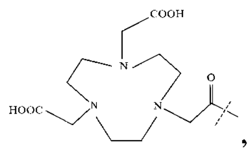

reacting Compound **4** with NOTA-NHS-ester to generate a compound represented by Formula I-1, and the reaction scheme is:

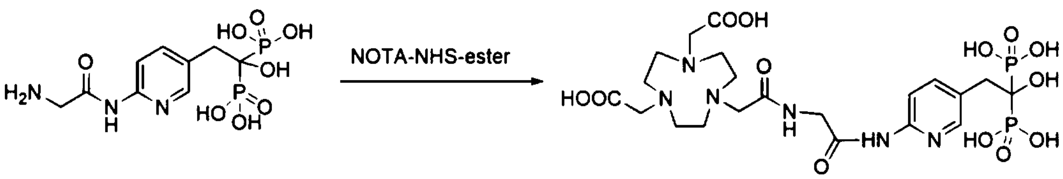


Compound **4**

Formula I-1

when $R_1$ is

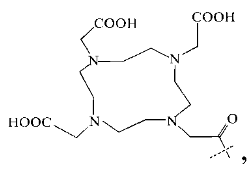

reacting Compound **4** with DOTA-NHS-ester to generate a compound represented by Formula I-2, and the reaction scheme is:

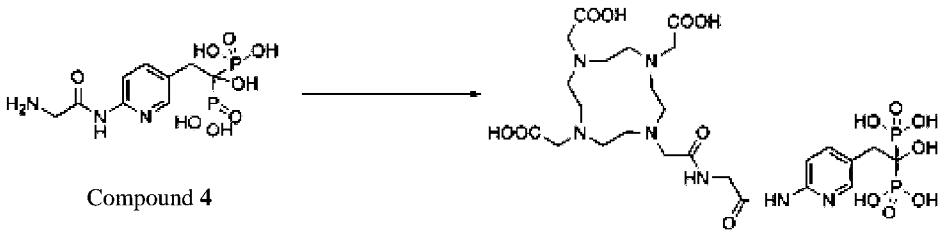

Compound 4

Formula I-2

,

preferably,

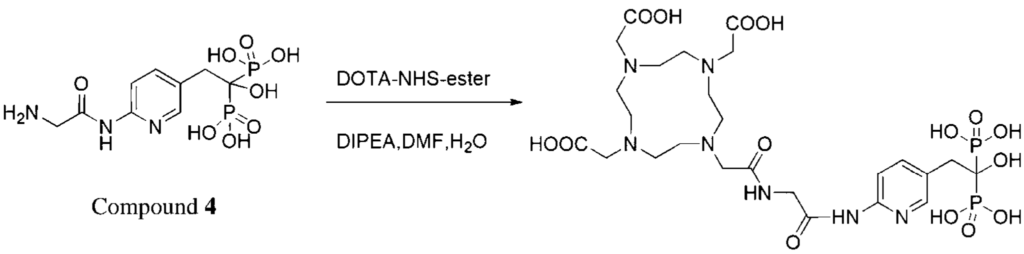


Compound 4

Formula I-2

when $R_1$ is

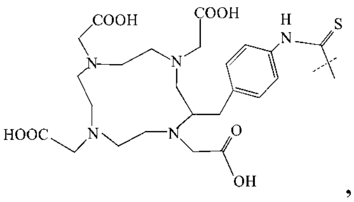

,

reacting Compound **4** with DOTA-p-Bn-NCS to generate a compound represented by Formula I-3, and the reaction scheme is:

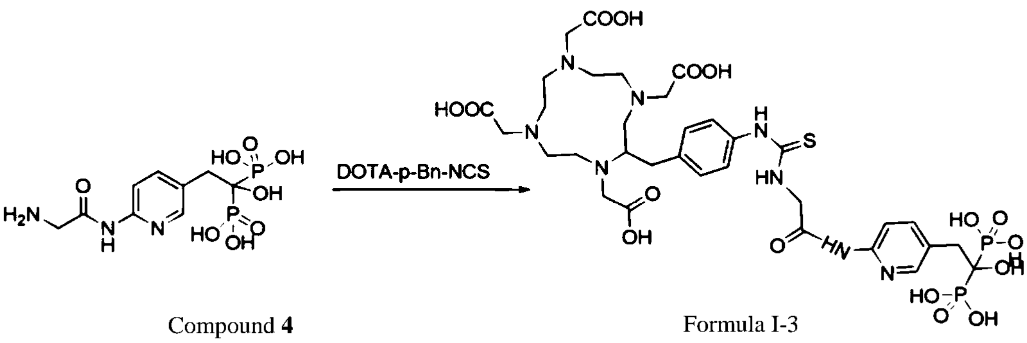


Compound **4**

Formula I-3

.

5. A method for preparing the radiolabeled compound according to claim 2 or 3, which comprises a step of reacting the compound represented by Formula I according to claim 1 with a radionuclide salt solution to obtain the radiolabeled compound represented by Formula II.

6. The method according to claim 5, which comprises steps of mixing a solution of the compound represented by Formula I according to claim 1, a sodium salt solution and the radionuclide salt solution, adjusting the pH value of the obtained mixed solution, reacting the obtained solution, adjusting the pH value of the resulting reaction product, and filtering the resulting product to obtain the radiolabeled compound represented by Formula II.

7. The method for preparing the radiolabeled compound according to claim 5 or 6, wherein, when the radionuclide is $^{68}$Ga, $^{111}$In, $^{89}$Zr, $^{177}$Lu or $^{64}$Cu, the method comprises steps of mixing a solution of the compound represented by Formula I according to claim 1, a sodium acetate solution and the radionuclide salt solution, adjusting pH value of the obtained mixed solution, reacting the obtained solution, adjusting pH value of the resulting reaction product, sterilizing the resulting product, and filtering to obtain the radiolabeled compound;

when the radionuclide is $^{225}$Ac, the method comprises steps of mixing a solution of the compound represented by Formula I according to claim 1, a sodium citrate solution, a sodium ascorbate solution and the radionuclide salt solution, adjusting pH value of the obtained mixed solution, reacting the obtained solution, adjusting pH value of the resulting reaction product, sterilizing the resulting product, and filtering to obtain the radiolabeled compound;
when the radionuclide is $^{211}$At, the method comprises steps of mixing a solution of the compound represented by Formula I according to claim 1, a sodium borate solution and the radionuclide salt solution, adjusting pH value of the obtained mixed solution, reacting the obtained solution, adjusting pH value of the resulting reaction product, sterilizing the resulting product, and filtering to obtain the radiolabeled compound.

8. The method for preparing the radiolabeled compound according to any one of claims 5 to 7, which comprises a step of reacting the compound represented by Formula I-1 with the radionuclide salt solution, specifically comprises the following steps:

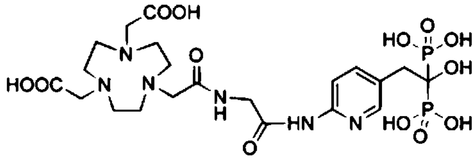

Formula I-1

when the radionuclide is $^{68}$Ga, adding 0.8-1.5ml of 0.25M sodium acetate solution to a solution of the compound represented by Formula I-1 with a solute content of 30-40μg, then adding a $^{68}$Ga salt solution with an activity of 10mCi, and mixing; adjusting the pH value of the obtained mixed solution to 4-7, preferably 5; reacting the obtained solution at 80-100°C, preferably 95°C, with a reaction time of 10-30min, preferably 15min; after the reaction, adjusting the pH value of the resulting reaction product to 5; wherein the solution of the compound represented by Formula I-1 has a concentration of 1mg/ml, and the $^{68}$Ga salt solution has a concentration of 5mCi/ml to 10mCi/ml;
when the radionuclide is $^{111}$In, adding 0.8-1.5ml of 0.25M sodium acetate solution to a solution of the compound represented by Formula I-1 with a solute content of 30-40μg, then adding a $^{111}$In salt solution with an activity of 10mCi, and mixing; adjusting the pH value of the obtained mixed solution to 4-7, preferably 5.5; reacting the obtained solution at 80-100°C, preferably 95°C, with a reaction time of 10-30min, preferably 15min; after the reaction, adjusting the pH value of the resulting reaction product to 5; wherein the solution of the compound represented by Formula I-1 has a concentration of 1mg/ml; and the $^{111}$In salt solution has a concentration of 10mCi/ml to 20mCi/ml;
when the radionuclide is $^{89}$Zr, adding 0.8-1.5ml of 0.25M sodium acetate solution to a solution of the compound represented by Formula I-1 with a solute content of 30-40μg, then adding a $^{89}$Zr salt solution with an activity of 2mCi, and mixing; adjusting the pH value of the obtained mixed solution to 4-7, preferably 5; reacting the obtained solution at 80-100°C, preferably 80°C, with a reaction time of 10-30min, preferably 15min; after the reaction, adjusting the pH value of the resulting reaction product to 4.5; wherein the solution of the compound represented by Formula I-1 has a concentration of 1mg/ml, and the $^{89}$Zr salt solution has a concentration of 10mCi/ml to 20mCi/ml;
when the radionuclide is $^{177}$Lu, adding 0.8-1.5 ml of 0.25M sodium acetate solution to a solution of the compound represented by Formula I-1 with a solute content of 30-40μg, then adding a $^{177}$Lu salt solution with an activity of 20mCi, and mixing; adjusting the pH value of the obtained mixed solution to 4-7, preferably 5; reacting the obtained solution at 80-100°C, preferably 95°C, with a reaction time of 10-30min, preferably 15min; after the reaction, adjusting the pH value of the resulting reaction product to 4.5; wherein the solution of the compound represented by Formula I-1 has a concentration of 1mg/ml, and the $^{177}$Lu salt solution has a concentration of 20mCi/ml to 30mCi/ml;
when the radionuclide is $^{225}$Ac, adding 0.8-1.5 ml of 0.1M sodium ascorbate solution and 0.8-1.5 ml of 0.1M sodium citrate solution to a solution of the compound represented by Formula I-1 with a solute content of 20-30μg,

then adding a $^{225}Ac$ salt solution with an activity of 0.01mCi, and mixing; adjusting the pH value of the obtained mixed solution to 4-7, preferably 5; reacting the obtained solution at 80-100°C, preferably 95°C, with a reaction time of 10-30min, preferably 15min; after the reaction, adjusting the pH value of the resulting reaction product to 5.5; wherein the solution of the compound represented by Formula I-1 has a concentration of 1mg/ml, and the $^{225}Ac$ salt solution has a concentration of 0.01mCi/ml to 0.02mCi/ml;

when the radionuclide is $^{64}Cu$, adding 0.8-1.5ml of 0.25M sodium acetate solution to a solution of the compound represented by Formula I-1 with a solute content of 30-40μg, then adding a $^{64}Cu$ salt solution with an activity of 5mCi, and mixing; adjusting the pH value of the obtained mixed solution to 4-7, preferably 5; reacting the obtained solution at 80-100°C, preferably 95°C, with a reaction time of 10-30min, preferably 15min; after the reaction, adjusting the pH value of the resulting reaction product to 4.5; wherein the solution of the compound represented by Formula I-1 has a concentration of 1mg/ml, and the $^{64}Cu$ salt solution has a concentration of 5mCi/ml to 10mCi/ml;

when the radionuclide is $^{211}At$, adding 0.8-1.5ml of 0.25M sodium borate solution to a solution of the compound represented by Formula I-1 with a solute content of 30-40μg, then adding a $^{211}At$ salt solution with an activity of 1mCi, and mixing; adjusting the pH value of the obtained mixed solution to 4-7, preferably 5; reacting the obtained solution at 80-100°C, preferably 95°C with a reaction time of 10-30min, preferably 15min; after the reaction, adjusting the pH value of the resulting reaction product to 6.5; wherein the solution of the compound represented by Formula I-1 has a concentration of 1mg/ml, and the $^{211}At$ salt solution has a concentration of 1.0mCi/ml to 2.0mCi/ml.

**9.** The method for preparing the radiolabeled compound according to any one of claims 5 to 7, which comprises a step of reacting the compound represented by Formula I-2 with the radionuclide salt solution, specifically comprises the following steps:

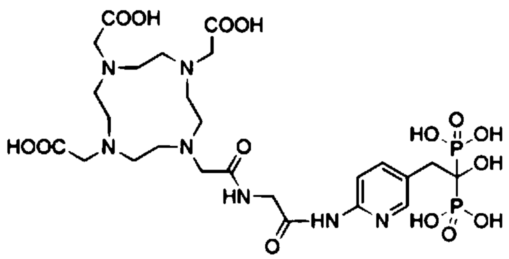

Formula I-2

when the radionuclide is $^{68}Ga$, adding 0.8-1.5ml of 0.25M sodium acetate solution to a solution of the compound represented by Formula I-2 with a solute content of 30-40μg, then adding a $^{68}Ga$ salt solution with an activity of 10mCi, and mixing; adjusting the pH value of the obtained mixed solution to 4-7, preferably 4.5; reacting the obtained solution at 80-100°C, preferably 95°C, with a reaction time of 10-30min, preferably 15min; after the reaction, adjusting the pH value of the resulting reaction product to 5; wherein the solution of the compound represented by Formula I-2 has a concentration of 1mg/ml, and the $^{68}Ga$ salt solution has a concentration of 5mCi/ml to 10mCi/ml;

when the radionuclide is $^{111}In$, adding 0.8-1.5ml of 0.25M sodium acetate solution to a solution of the compound represented by Formula I-2 with a solute content of 30-40μg, and then adding a $^{111}In$ salt solution with an activity of 10mCi, and mixing; adjusting the pH value of the obtained mixed solution to 4-7, preferably 5.5; reacting the obtained solution at 80-100°C, preferably 95°C, with a reaction time of 10-30min, preferably 15min; after the reaction, adjusting the pH value of the resulting reaction product to 4.5; wherein the solution of the compound represented by Formula I-2 has a concentration of 1mg/ml, and the $^{111}In$ salt solution has a concentration of 10mCi/ml to 20mCi/ml;

when the radionuclide is $^{89}Zr$, adding 0.8-1.5ml of 0.25M sodium acetate solution to a solution of the compound represented by Formula I-2 with a solute content of 30-40μg, and then adding a $^{89}Zr$ salt solution with an activity of 2mCi, and mixing; adjusting the pH value of the obtained mixed solution to 4-7, preferably 5; reacting the obtained solution at 80-100°C, preferably 80°C, with a reaction time of 10-30 min, preferably 15 min; after the reaction, adjusting the pH value of the resulting reaction product to 4.5; wherein the solution of the compound represented by Formula I-2 has a concentration of 1 mg/ml, and the $^{89}Zr$ salt solution has a concentration of 10 mCi/ml to 20 mCi/ml;

when the radionuclide is $^{177}Lu$, adding 0.8-1.5 ml of 0.25 M sodium acetate solution to a solution of the compound represented by Formula I-2 with a solute content of 30-40 μg, and then adding a $^{177}Lu$ salt solution with an activity

of 20 mCi, and mixing; adjusting the pH value of the obtained mixed solution to 4-7, preferably 5; reacting the obtained solution at 80-100°C, preferably 85°C, with a reaction time of 10-30min, preferably 15min; after the reaction, adjusting the pH value of the resulting reaction product to 5; wherein the solution of the compound represented by Formula I-2 has a concentration of 1mg/ml, and the $^{177}$Lu salt solution has a concentration of 20mCi/ml to 30mCi/ml;

when the radionuclide is $^{225}$Ac, adding 0.8-1.5ml of 0.1M sodium ascorbate solution and 0.8-1.5 ml of 0.1 M sodium citrate solution to a solution of the compound represented by Formula I-2 with a solute content of 20-30μg, then adding a $^{225}$Ac salt solution with an activity of 0.01mCi, and mixing; adjusting the pH value of the obtained mixed solution to 4-7, preferably 5; reacting the obtained solution at 80-100°C, preferably 90°C, with a reaction time of 10-30min, preferably 15min; after the reaction, adjusting the pH value of the resulting reaction product to 5.5; wherein the solution of the compound represented by Formula I-2 has a concentration of 1mg/ml, and the $^{225}$Ac salt solution has a concentration of 0.01mCi/ml to 0.02mCi/ml;

when the radionuclide is $^{64}$Cu, adding 0.8-1.5 ml of 0.25 M sodium acetate solution to a solution of the compound represented by Formula I-2 with a solute content of 30-40μg, then adding a $^{64}$Cu salt solution with an activity of 5 mCi, and mixing; adjusting the pH value of the obtained mixed solution to 4-7, preferably 5.5; reacting the obtained solution at 80-100°C, preferably 90°C, with a reaction time of 10-30 min, preferably 15 min; after the reaction, adjusting the pH value of the resulting reaction product to 4.5; wherein the solution of the compound represented by Formula I-2 has a concentration of 1 mg/ml, and the $^{64}$Cu salt solution has a concentration of 5mCi/ml to 10mCi/ml;

when the radionuclide is $^{211}$At, adding 0.8-1.5ml of 0.25M sodium borate solution to a solution of the compound represented by Formula I-2 with a solute content of 30-40μg, then adding a $^{211}$At salt solution with an activity of 1mCi, and mixing; adjusting the pH value of the obtained mixed solution to 4-7, preferably 5; reacting the obtained solution at 80-100°C, preferably 95°C, with a reaction time of 10-30min, preferably 15min; after the reaction, adjusting the pH value of the resulting reaction product to 6.5; wherein the solution of the compound represented by Formula I-2 has a concentration of 1mg/ml, and the $^{211}$At salt solution has a concentration of 1.0mCi/ml to 2.0mCi/ml.

**10.** The method for preparing the radiolabeled compound according to any one of claims 5 to 7, which comprises a step of reacting the compound represented by Formula I-3 with the radionuclide salt solution, specifically comprises the following steps:

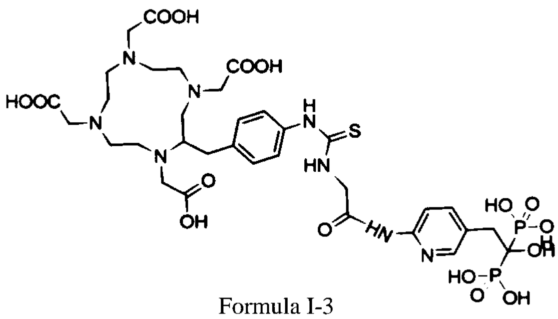

Formula I-3

when the radionuclide is $^{68}$Ga, adding 0.8-1.5ml of 0.25M sodium acetate solution to a solution of the compound represented by Formula I-3 with a solute content of 20-40μg, then adding a $^{68}$Ga salt solution with an activity of 10mCi, and mixing; adjusting the pH value of the obtained mixed solution to 4-7, preferably 5; reacting the obtained solution at 80-100°C, preferably 85°C, with a reaction time of 10-30min, preferably 15min; after the reaction, adjusting the pH value of the resulting reaction product to 5; wherein the solution of the compound represented by Formula I-3 has a concentration of 1mg/ml, and the $^{68}$Ga salt solution has a concentration of 5mCi/ml to 10mCi/ml;

when the radionuclide is $^{111}$In, adding 0.7-1.4ml of 0.25M sodium acetate solution to a solution of the compound represented by Formula I-3 with a solute content of 30-40μg, then adding a $^{111}$In salt solution with an activity of 10mCi, and mixing; adjusting the pH value of the obtained mixed solution to 4-7, preferably 5.5; reacting the obtained solution at 80-100°C, preferably 90°C, with a reaction time of 10-30 min, preferably 15min; after the reaction, adjusting the pH value of the resulting reaction product to 5; wherein the solution of the compound represented by Formula I-3 has a concentration of 1mg/ml, and the $^{111}$In salt solution has a concentration of 10mCi/ml to 20mCi/ml;

when the radionuclide is $^{89}$Zr, adding 0.8-1.5ml of 0.25M sodium acetate solution to a solution of the compound

represented by Formula I-3 with a solute content of 30-40μg, then adding a $^{89}Zr$ salt solution with an activity of 2mCi, and mixing; adjusting the pH value of the obtained mixed solution to 4-7, preferably 5; reacting the obtained solution at 80-100°C, preferably 80°C, with a reaction time of 10-30min, preferably 15min; after the reaction, adjusting the pH value of the resulting reaction product to 4.5; wherein the solution of the compound represented by Formula I-3 has a concentration of 1mg/ml, and the $^{89}Zr$ salt solution has a concentration of 10mCi/ml to 20mCi/ml;

when the radionuclide is $^{177}Lu$, adding 0.8-1.5ml of 0.25M sodium acetate solution to a solution of the compound represented by Formula I-3 with a solute content of 30-40μg, then adding a $^{177}Lu$ salt solution with an activity of 20mCi, and mixing; adjusting the pH value of the obtained mixed solution to 4-7, preferably 5; reacting the obtained solution at 80-100°C, preferably 95°C, with a reaction time of 10-30min, preferably 15min; after the reaction, adjusting the pH value of the resulting reaction product to 4.5; wherein the solution of the compound represented by Formula I-3 has a concentration of 1mg/ml, and the $^{177}Lu$ salt solution has a concentration of 20mCi/ml to 30mCi/ml;

when the radionuclide is $^{225}Ac$, adding 0.8-1.5ml of 0.1M sodium ascorbate solution and 0.8-1.5ml of 0.1M sodium citrate solution to a solution of the compound represented by Formula I-3 with a solute content of 20-30μg, then adding a $^{225}Ac$ salt solution with an activity of 0.01mCi, and mixing; adjusting the pH value of the obtained mixed solution to 4-7, preferably 5; reacting the obtained solution at 80-100°C, preferably 95°C, with a reaction time of 10-30min, preferably 15min; after the reaction, adjusting the pH value of the resulting reaction product to 5.5; wherein the solution of the compound represented by Formula I-3 has a concentration of 1mg/ml, and the $^{225}Ac$ salt solution has a concentration of 0.01mCi/ml to 0.02mCi/ml;

when the radionuclide is $^{64}Cu$, adding 0.8-1.5 ml of 0.25M sodium acetate solution to a solution of the compound represented by Formula I-3 with a solute content of 30-40μg, then adding a $^{64}Cu$ salt solution with an activity of 5 mCi, and mixing; adjusting the pH value of the obtained mixed solution to 4-7, preferably 5; reacting the obtained solution at 80-100°C, preferably 95°C, with a reaction time of 10-30 min, preferably 15 min; after the reaction, adjusting the pH value of the resulting reaction product to 4.5; wherein the solution of the compound represented by Formula I-3 has a concentration of 1 mg/ml, and the $^{64}Cu$ salt solution has a concentration of 5 mCi/ml to 10 mCi/ml;

when the radionuclide is $^{211}At$, adding 0.8-1.5 ml of 0.25 M sodium borate solution to a solution of the compound represented by Formula I-3 with a solute content of 30-40μg, then adding a $^{211}At$ salt solution with an activity of 1 mCi, and mixing; adjusting the pH value of the obtained mixed solution to 4-7, preferably 5; reacting the obtained solution at 80-100°C, preferably 95°C, with a reaction time of 10-30 min, preferably 15 min; after the reaction, adjusting the pH value of the resulting reaction product to 6.5; wherein the solution of the compound represented by Formula I-3 has a concentration of 1 mg/ml, and the $^{211}At$ salt solution has a concentration of 1.0 mCi/ml to 2.0 mCi/ml.

**11.** Use of the precursor compound or pharmaceutically acceptable salt thereof according to claim 1 or the radiolabeled compound or pharmaceutically acceptable salt thereof according to claim 2 or 3 in the manufacture of a medicament for imaging and/or treating a metastatic bone tumor.

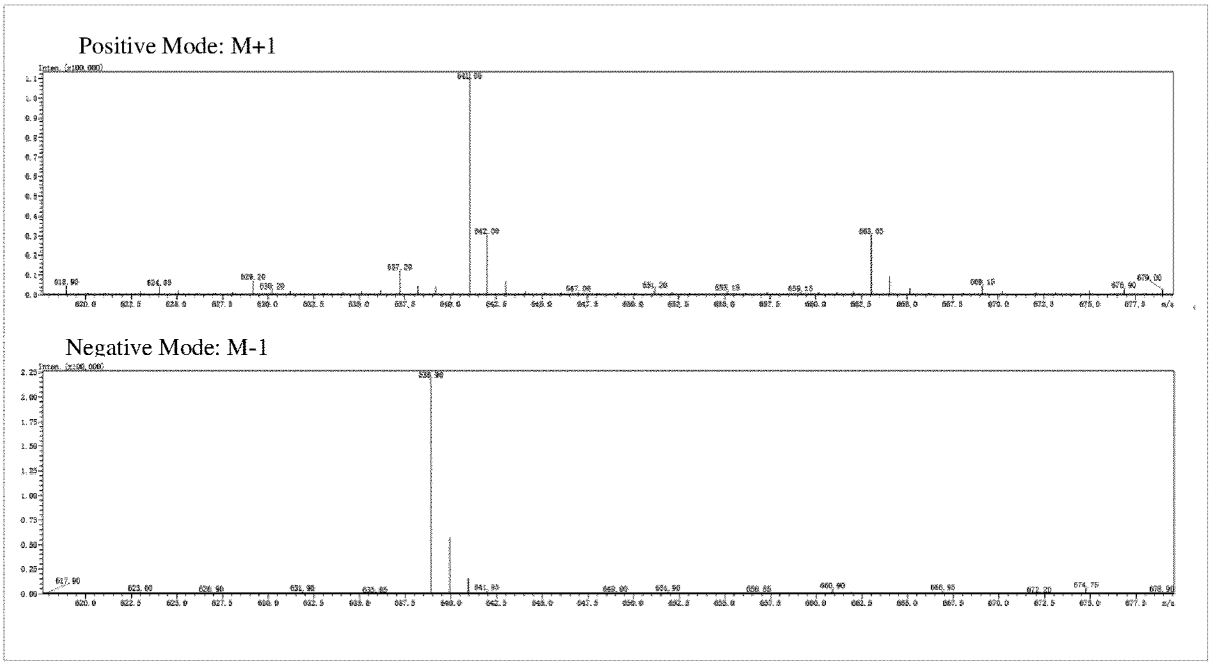


**Figure 1**

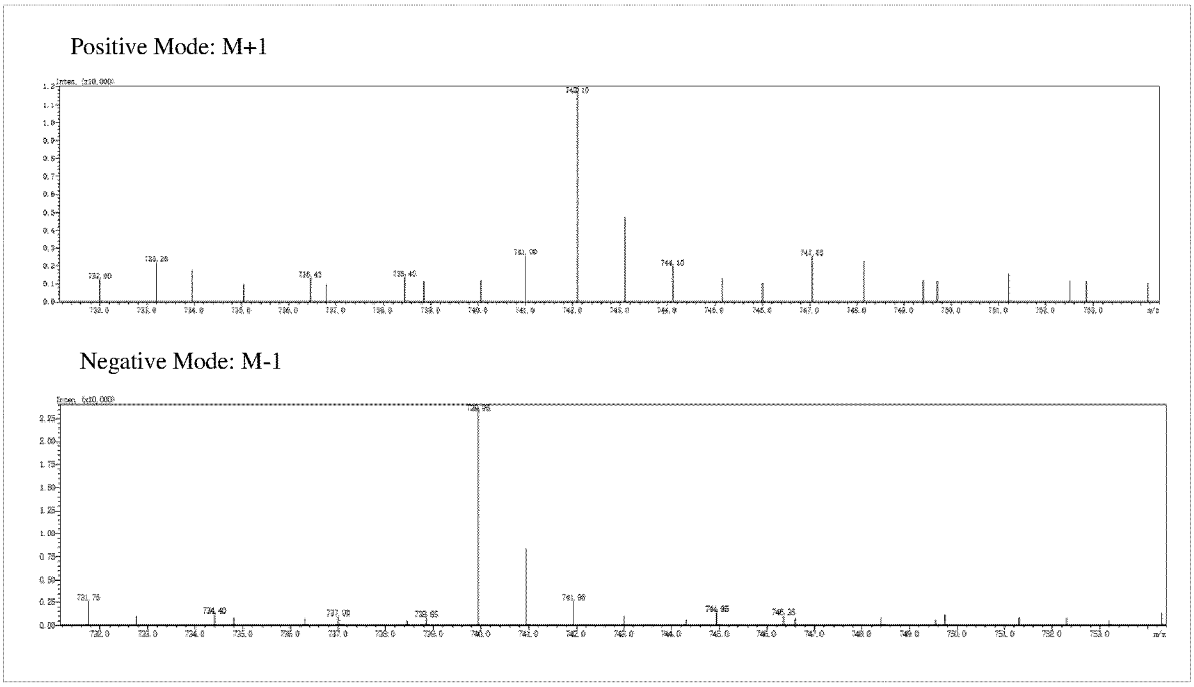


**Figure 2**

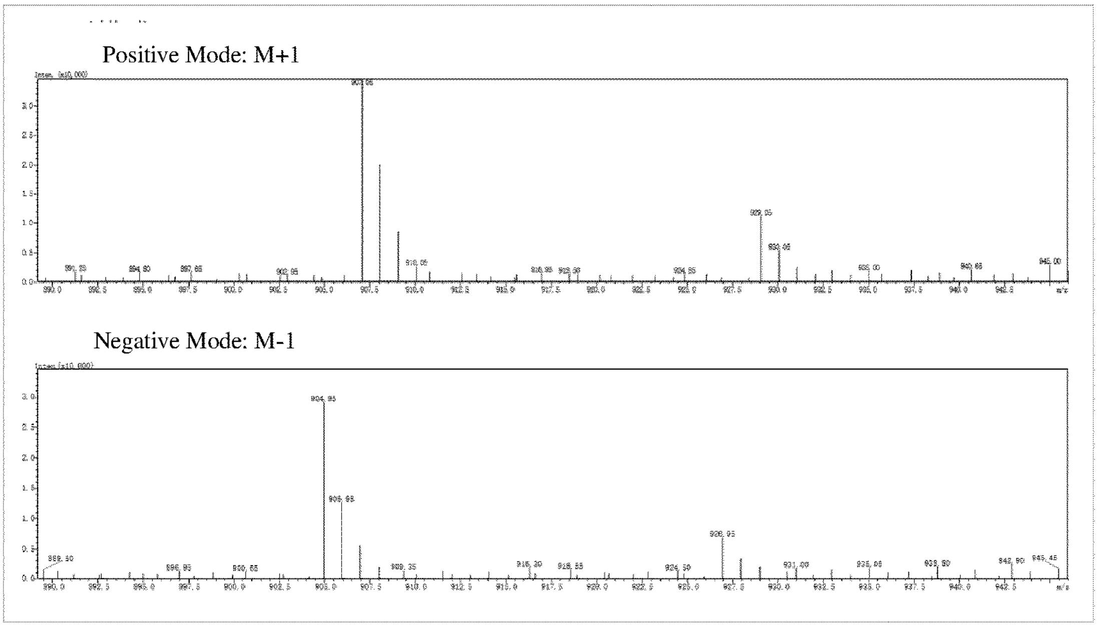


Figure 3

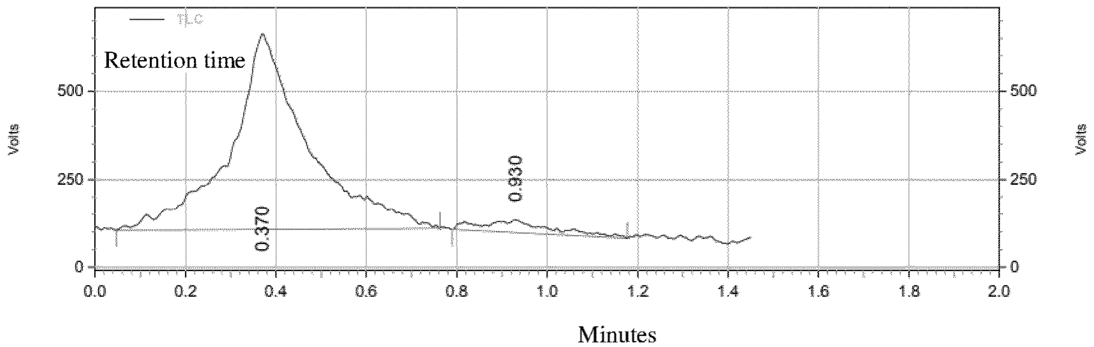


Figure 4

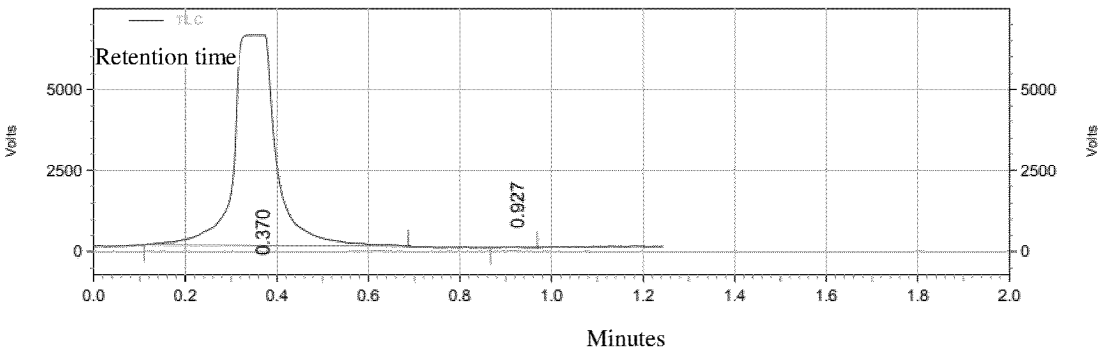


Figure 5

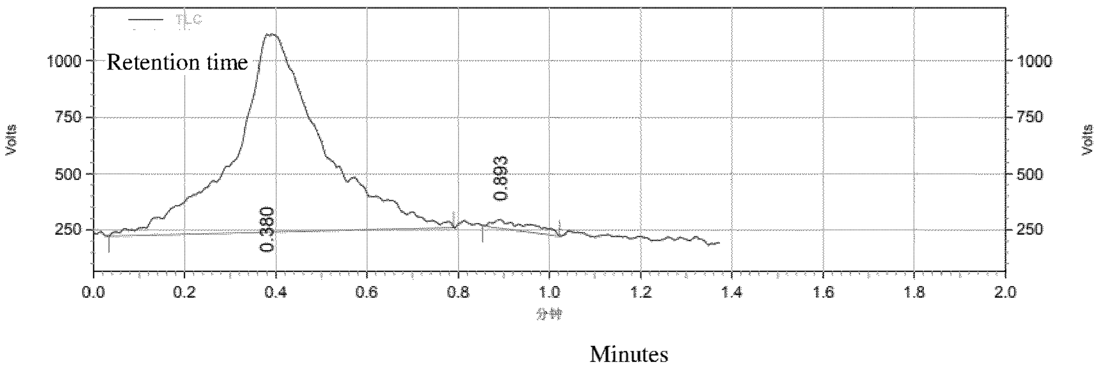


**Figure 6**

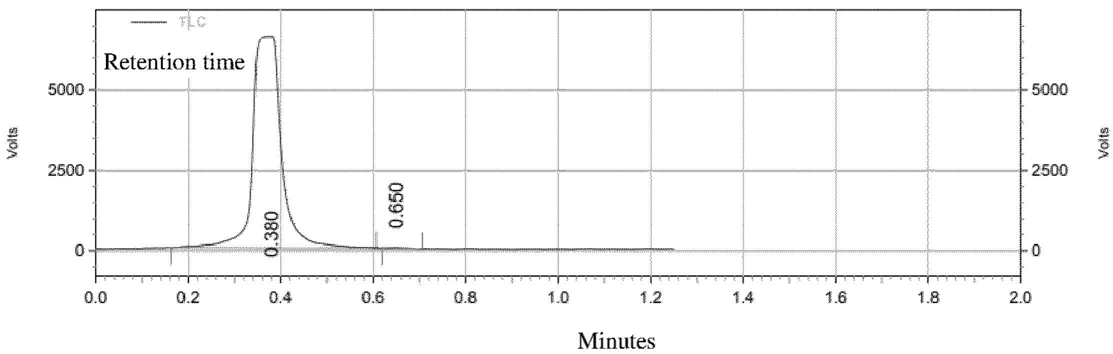


**Figure 7**

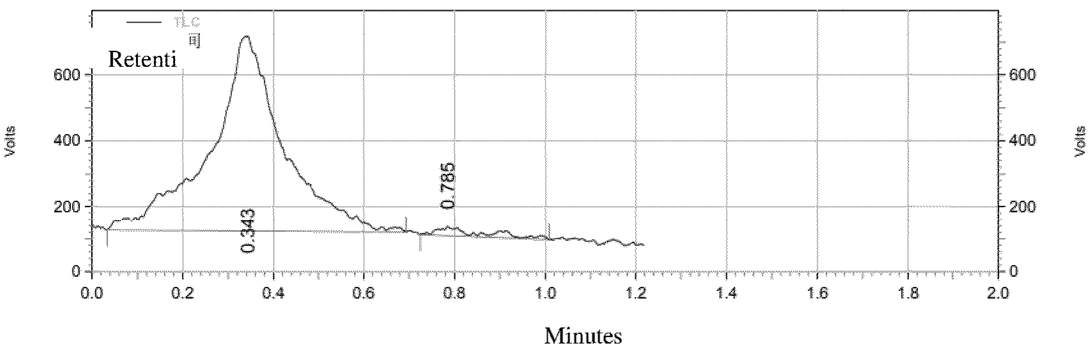


**Figure 8**

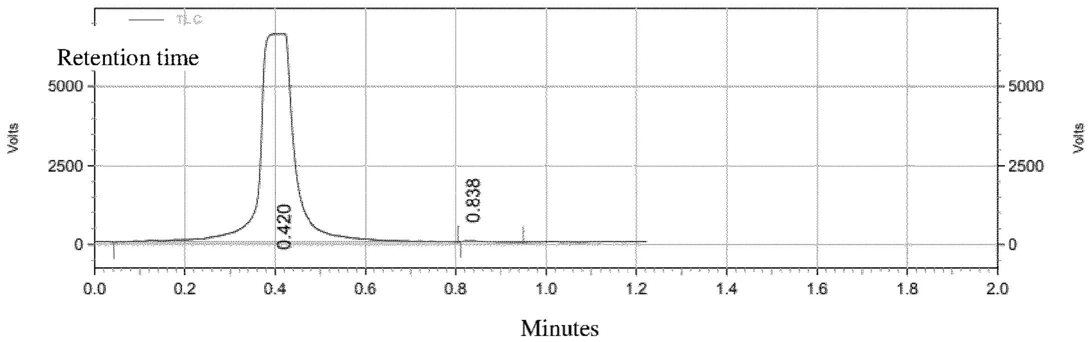


**Figure 9**

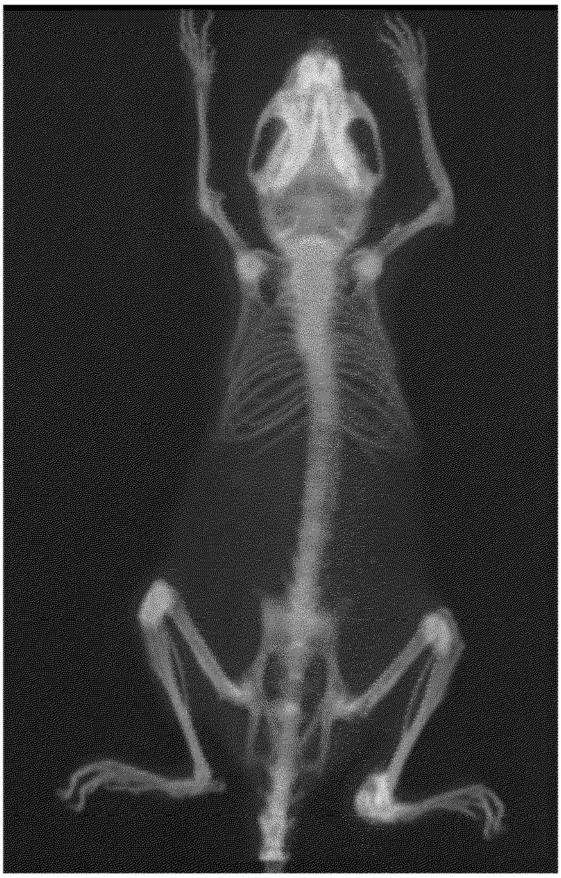

**Figure 10**

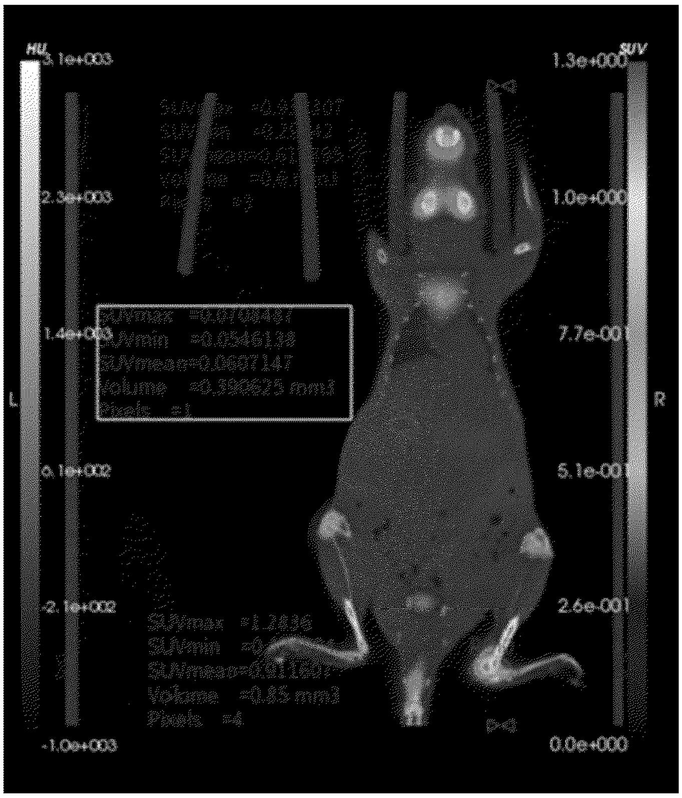
HU
3.1e+003
2.3e+003
1.4e+003
6.1e+002
-2.1e+002
-1.0e+003
SUV
1.3e+000
1.0e+000
7.7e-001
5.1e-001
2.6e-001
0.0e+000
L
R
SUVmin =0.0546138
SUVmean=0.0607147
Volume =0.390625 mm3
Pixels =1
SUVmax =1.2836
Volume =0.85 mm3
Pixels =4

Figure 11

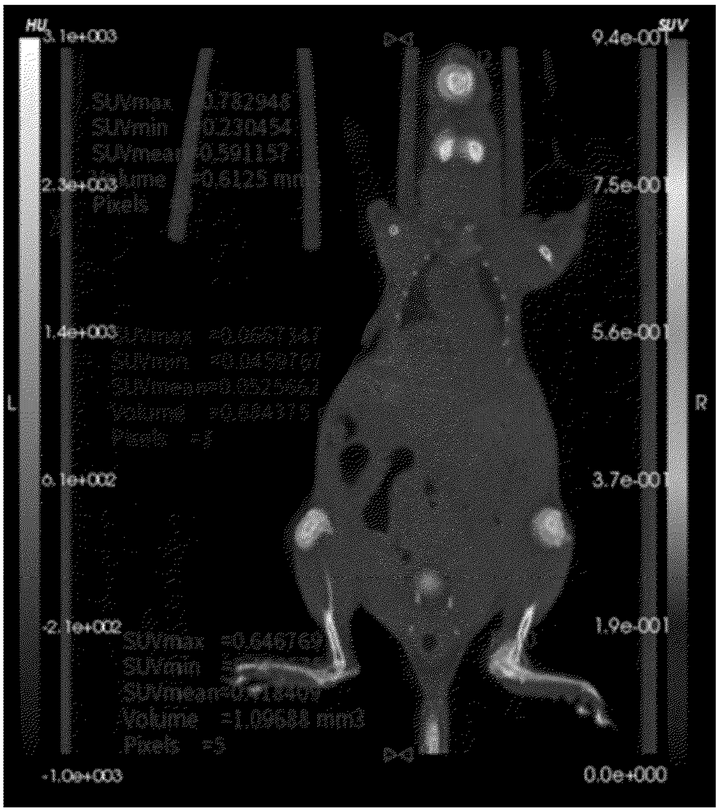

HU
3.1e+003
2.3e+003
1.4e+003
6.1e+002
-2.1e+002
-1.0e+003
SUV
9.4e-001
7.5e-001
5.6e-001
3.7e-001
1.9e-001
0.0e+000
L
R
SUVmax
SUVmin
SUVmean
Pixels
Volume =1.09688 mm3
Pixels =5


Figure 12

**INTERNATIONAL SEARCH REPORT**

International application No.
**PCT/CN2024/076669**

**A. CLASSIFICATION OF SUBJECT MATTER**

C07F 9/6558(2006.01)i; A61K 51/04(2006.01)i; A61K 31/675(2006.01)i; C07B 59/00(2006.01)i; A61P 35/04(2006.01)i; A61K 103/00(2006.01)i; A61K 103/20(2006.01)i; A61K 103/30(2006.01)i; A61K 103/40(2006.01)i

According to International Patent Classification (IPC) or to both national classification and IPC

**B. FIELDS SEARCHED**

Minimum documentation searched (classification system followed by classification symbols)

IPC:C07F,A61K,C07B,A61P

Documentation searched other than minimum documentation to the extent that such documents are included in the fields searched

Electronic data base consulted during the international search (name of data base and, where practicable, search terms used)

CNTXT, CNKI, WPABS, CNABS, REGISTRY(STN), CAPLUS(STN): 西南医科大学附属医院, 利噻膦酸, 利塞膦酸, 膦酸, 发射, 标记, risedronic acid, thiencarbazone-methyl phosphonic acid, radioactive, label, cas rn, 结构式检索, structural formula search

**C. DOCUMENTS CONSIDERED TO BE RELEVANT**

| Category* | Citation of document, with indication, where appropriate, of the relevant passages | Relevant to claim No. |
|---|---|---|
| PX | CN 116120367 A (AFFILIATED HOSPITAL OF SOUTHWEST MEDICAL UNIVERSITY) 16 May 2023 (2023-05-16)<br>claims 1-10 | 1-11 |
| A | CN 114230610 A (AFFILIATED HOSPITAL OF SOUTHWEST MEDICAL UNIVERSITY) 25 March 2022 (2022-03-25)<br>description, paragraphs [0005]-[0039] | 1-11 |
| A | CN 113372285 A (AFFILIATED HOSPITAL OF SOUTHWEST MEDICAL UNIVERSITY) 10 September 2021 (2021-09-10)<br>claims 1-10 | 1-11 |
| A | WO 2022216084 A1 (SEOUL NATIONAL UNIVERSITY R&DB FOUNDATION) 13 October 2022 (2022-10-13)<br>claims 1-5 | 1-11 |

☐ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **11 April 2024** | **19 April 2024** |
| Name and mailing address of the ISA/CN<br>**China National Intellectual Property Administration (ISA/CN)**<br>**China No. 6, Xitucheng Road, Jimenqiao, Haidian District, Beijing 100088** | Authorized officer<br><br>Telephone No. |

Form PCT/ISA/210 (second sheet) (July 2022)

**INTERNATIONAL SEARCH REPORT**
**Information on patent family members**

International application No.
**PCT/CN2024/076669**

| Patent document cited in search report | Publication date (day/month/year) | Patent family member(s) | Publication date (day/month/year) |
| --- | --- | --- | --- |
| CN 116120367 A | 16 May 2023 | None | |
| CN 114230610 A | 25 March 2022 | None | |
| CN 113372285 A | 10 September 2021 | None | |
| WO 2022216084 A1 | 13 October 2022 | KR 102366189 B1 | 23 February 2022 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 202310140015